(19) Europäisches Patentamt
European Patent Office
Office européen des brevets

(11) EP 0 477 001 B1

(12) **EUROPEAN PATENT SPECIFICATION**

(45) Date of publication and mention of the grant of the patent:
**26.06.1996 Bulletin 1996/26**

(51) Int Cl.[6]: **C12Q 1/32**, C12Q 1/02

(21) Application number: **91308520.5**

(22) Date of filing: **18.09.1991**

(54) **Highly sensitive assay method for myo-inositol and composition for practicing same**

Hochsensitives Testverfahren für Myo-Inositol und Komposition zur Ausführung davon

Méthode d'essai très sensitive pour myo-inositol et décomposition pour exercer la même

(84) Designated Contracting States:
**AT BE CH DE DK ES FR GB GR IT LI LU NL SE**

(30) Priority: **18.09.1990 JP 249775/90**
**18.09.1990 JP 249776/90**

(43) Date of publication of application:
**25.03.1992 Bulletin 1992/13**

(60) Divisional application: **95110124.5**

(73) Proprietor: **ASAHI KASEI KOGYO KABUSHIKI KAISHA**
**Osaka (JP)**

(72) Inventors:
- **Ueda, Shigeru**
  **Tagata-gun, Shizuoka (JP)**
- **Takahashi, Mamoru**
  **Sunto-gun, Shizuoka (JP)**
- **Misaki, Hideo**
  **Tagata-gun, Shizuoka (JP)**
- **Imamura, Shigeyuki**
  **Tagata-gun, Shizuoka (JP)**
- **Matsuura, Kazuo**
  **Tagata-gun, Shizuoka (JP)**

(74) Representative: **Woods, Geoffrey Corlett et al**
**J.A. KEMP & CO.**
**14 South Square**
**Gray's Inn**
**London WC1R 5LX (GB)**

(56) References cited:
- **JOURNAL OF BIOLOGICAL CHEMISTRY, vol. 254, no. 16, 1979, Washington, DC (US); R. RAMELEY et al., pp. 7684-7690**
- **ANALYTICAL BIOCHEMISTRY, vol. 158, no. 1, 01 October 1986, New York, NY (US); T.W. GUDERMAN et al., pp. 59-63**
- **ANALYTICA CHIMICA ACTA, vol. 206, nos. 1-2, 1988, Amsterdam (NL); B. OLSSON et al., pp. 49-55**
- **ANALYTICAL BIOCHEMISTRY, vol. 141, no. 2, 1984, New York, NY (US); L. MacGREGOR et al., pp. 382-389**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to a highly sensitive method of assaying a specimen for myo-inositol and to a composition suitable for assaying myo-inositol.

Myo-inositol is one of nine isomers of inositol and is a stable cyclic alcohol. In humans myo-inositol is supplied in an amount of approximately one gram per day from daily meals and approximately 2 grams from renal biosynthesis.

The blood level of myo-inositol is controlled to a constant level by a balance of intake into cells and renal excretion, reabsorption and oxidation.

The plasma level of myo-inositol is increased by a renal functional disorder [Clinical Chem., 24(11); 1448-1455 (1988)]. The renal function can be monitored by measuring the level of myo-inositol in the blood.

Heretobefore the amount of myo-inositol in a specimen has been measured by gas-chromatography and high performance liquid chromatography (HPLC). These measuring methods have a number of disadvantages, for example the specimen has to be previously treated and the operation needs a complex treatment. Hence large numbers of assays for clinical chemistry was impossible. The normal standard level of myo-inositol in blood plasma is quite low, such as approximately 30 µmol/l [Proceedings of Japan Clinical Chemistry Annual Meeting No. 28 (1988)]. A simple and highly sensitive assay method is desired.

An enzymatic cycling method for assaying trace amount of substrates or enzyme activity using two types of enzyme to amplify the sensitivity is known. Among them, NAD-cycling, CoA-cycling and ATP-cycling are known but are almost never applied to routine assay methods for clinical chemistry due to complex operations.

We have found that myo-inositol dehydrogenase (EC. 1.1.1.18) can be used in a cycling reaction with thio-NADP group coenzymes.

The present invention provides a method of assaying a specimen for myo-inositol which comprises reacting the specimen with:

a) myo-inositol dehydrogenase using one of the thionictoinamide adenine dinucleotide phosphate group (thio-NADP group) or thionicotinamide adenine dinucleotide group (thio-NAD group) and one of the nicotinamide adenine dinucleotide phosphate group (NADP group) or nicotinamide adenine dinucleotide group (NAD group) as coenzymes, which myo-inositol dehydrogenase catalyzes a reversible reaction forming myo-inosose from myo-inositol,
b) $A_1$, and
c) $B_1$;
to effect the cycling reaction:

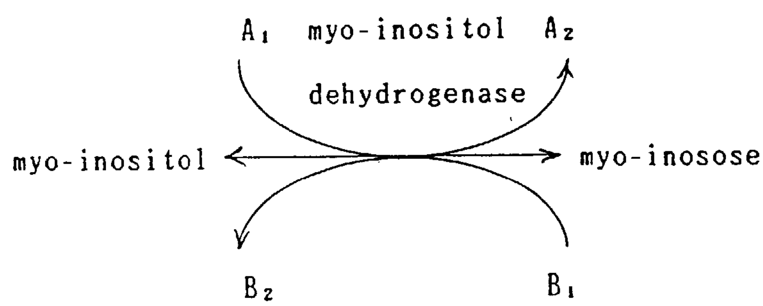


wherein:

$A_1$ is a thio-NADP, thio-NAD, NADP or NAD group compound,
$A_2$ is a reduced form of $A_1$,
$B_1$ is a reduced NADP or reduced NAD group compound when $A_1$ is a thio-NADP or thio-NAD group compound or $B_1$ is a reduced thio-NADP or reduced thio-NAD group compound when $A_1$ is a NADP or NAD group compound, and
$B_2$ is an oxidized form of $B_1$; and

measuring the amount of $A_2$ or $B_1$ generated or consumed in the cycling reaction.

The present invention also provides a method of assaying a specimen for myo-inositol which comprises reacting the specimen with:

a) myo-inositol dehydrogenase using one of the thio-NADP group or thio-NAD group and one of the NADP group

or NAD group as coenzymes, which myo-inositol dehydrogenase catalyzes a reversible reaction forming myo-inosose from myo-inositol,

b) $A_1$,

c) $B_1$ or/and $B_2$, and

d) a dehydrogenase (dehydrogenase X) which does not act on myo-inositol and which catalyses a reaction forming $B_1$ from $B_2$, and a substrate for the said dehydrogenase X;

to effect the cycling reaction:

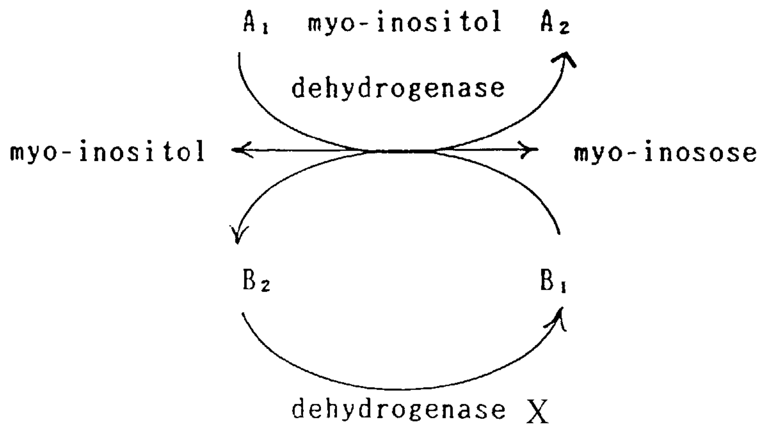


wherein:

$A_1$ is a thio-NADP, thio-NAD, NADP or NAD group compound,

$A_2$ is a reduced form of $A_1$,

$B_1$ is a reduced NADP or reduced NAD group compound when $A_1$ is a thio-NADP or thio-NAD group compound or $B_1$ is a reduced thio-NADP or reduced thio-NAD group compound when $A_1$ is a NADP or NAD group compound, and

$B_2$ is an oxidized form of $B_1$;

the reaction from $B_2$ to $B_1$ being an enzymatic reaction which generates $B_1$ from $B_2$ by an action of the dehydrogenase X with a coenzyme; and

measuring the amount of $A_2$ generated in the cycling reaction.

The present invention further provides a method of assaying a specimen for myo-inositol which comprises reacting the specimen with:

a) myo-inositol dehydrogenase using one of the thio-NADP group or thio-NAD group and one of the NADP group or NAD group as coenzymes, which myo-inositol dehydrogenase catalyzes a reversible reaction forming myo-inosose from myo-inositol,

b) $A_1$ or/and $A_2$,

c) $B_1$ and

d) a dehydrogenase (dehydrogenase Y) which does not act on myo-inositol and which catalyses a reaction from $A_2$ to $A_1$, and a substrate for the said dehydrogenase Y;

to effect the cycling reaction:

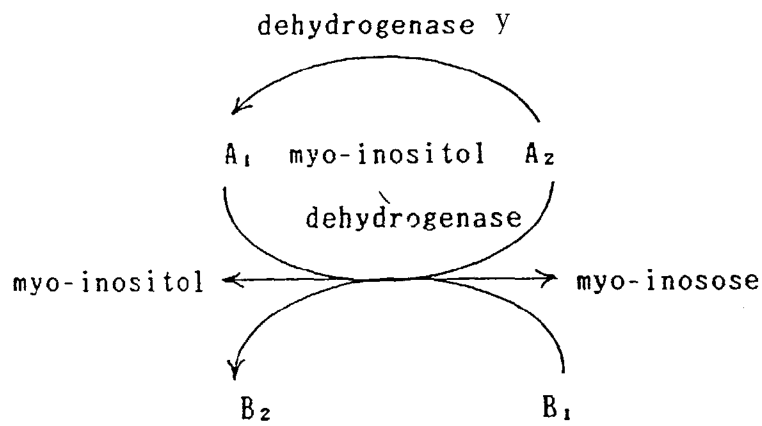


wherein:

$A_1$ is a thio-NADP, thio-NAD, NADP or NAD group compound,
$A_2$ is a reduced form of $A_1$,
$B_1$ is a reduced NADP or reduced NAD group compound when $A_1$ is a NADP or NAD group compound, and
$B_2$ is an oxidized form of $B_1$;
the reaction from $A_2$ to $A_1$ being an enzymatic reaction which generates $A_1$ from $A_2$ by an action of the dehydrogenase Y with a coenzyme; and

measuring the amount of $B_1$ consumed in the cycling reaction.

The present invention additionally provides a reagent composition suitable for assaying myo-inositol which comprises:

a) myo-inositol dehydrogenase using one of the thionicotinamide adenine dinucleotide phosphate (thio-NADP group) or thionicotinamide adenine dinucleotide group (thio-NAD group) and one of the nicotinamide adenine dinucleotide phosphate group (NADP group) or nicotinamide adenine dinucleotide group (NAD group) as coenzymes, which myo-inositol dehydrogenase catalyzes a reversible reaction forming myo-inosose from myo-inositol,
b) $A_1$ and
c) $B_1$;

wherein:

$A_1$ is a thio-NADP, thio-NAD, NADP or NAD group compound,
$B_1$ is a reduced NADP or reduced NAD group compound when $A_1$ is a thio-NADP or thio-NAD compound or $B_1$ is a reduced thio-NADP or reduced thio-NAD group compound when $A_1$ is a NADP or NAD group compound.

The present invention also provides a reagent composition suitable for assaying myo-inositol which comprises:

a) myo-inositol dehydrogenase using one of the thio-NADP group or thio-NAD group and one of the NADP group or NAD group as coenzymes, which myo-inositol dehydrogenase catalyzes a reversible reaction forming myo-inosose from myo-inositol,
b) $A_1$,
c) $B_1$ or/and $B_2$, and
d) a dehydrogenase (dehydrogenase X) which does not act on myo-inositol and which catalyzes a reaction forming $B_1$ from $B_2$, and a substrate for the said dehydrogenase X,

wherein:

$A_1$ is a thio-NADP, thio-NAD, NADP or NAD group compound,
$B_1$ is a reduced NADP or reduced NAD group compound when $A_1$ is a thio-NADP or thio-NAD group compound or $B_1$ is a reduced thio-NADP or reduced thio-NAD group compound when $A_1$ is a NADP or NAD group compound, and

$B_2$ is an oxidized form of $B_1$.

The present invention further provides a reagent composition suitable for assaying myo-inositol which comprises:

a) myo-inositol dehydrogenase using one of the thio-NADP group or thio-NAD group and one of the NADP group or NAD group as coenzymes, which myo-inositol dehydrogenase catalyzes a reversible reaction forming myo-inosose from myo-inositol,
b) $A_1$ or/and $A_2$
c) $B_1$ and
d) a dehydrogenase (dehydrogenase Y) which does not act on myo-inositol and which catalyzes a reaction from $A_2$ to $A_1$, and a substrate for the said dehydrogenase Y;

wherein:

$A_1$ is thio-NADP, thio-NAD, NADP or NAD group compound,
$B_1$ is a reduced NADP or reduced NAD group compound when $A_1$ is a thio-NADP or thio-NAD group compound or $B_1$ is a reduced thio-NADP or reduced thio-NAD group compound when $A_1$ is a NADP or NAD group compound, and
$A_2$ is an oxidized form of $A_1$.

The myo-inositol dehydrogenase has substrate specificity for myo-inositol and catalyses the reaction:

$$\text{myo-inositol} + \text{NAD} \rightleftharpoons \text{myo-inosose} + \text{reduced NADH}$$

The myo-inositol dehydrogenase may be produced by culturing a myo-inositol dehydrogenase producing microorganism belonging to genus Bacillus, and isolating myo-inositoldehydrogenase from the cultured mass.

This microorganism may be Bacillus sp. No. 3 (FERM BP-3013) or a mutant thereof which is capable of producing a myo-inositol dehydrogenase.

The assay method of the present invention depends on the difference in the maximum absorption of a reduced thio-NAD(P) group, which is an analogue of NAD(P), at approximately 400 nm, and that of a reduced NAD(P) group at approximately 340 nm. In the enzymatic cycling reaction using myo-inositol dehydrogenase, a thio-NAD(P) group is used as one of the two coenzymes, and the change in amount of any of the coenzymes is measured, for example by the difference in maximum absorptia of both reduced coenzymes. Thus the amount of myo-inositol in a specimen can be most precisely measured.

Figure 1 shows the heat stability of the novel myo-inositol dehydrogenase.

Figure 2 shows the optimum temperature of the myo-inositol dehydrogenase.

Figure 3 shows the pH-stability of the myo-inositol dehydrogenase.

Figure 4 shows the optimum pH of the myo-inositol dehydrogenase.

Figures 5 to 8 show assay curves of myo-inositol.

In the present invention, any type of myo-inositol dehydrogenase can be used so long as it has the above properties. Examples of myo-inositol dehydrogenase are described in the Enzyme Handbook (Asakura Publishing Co., Tokyo) and are produced by the following microorganisms:

Aerobacter aerogenes [J. Biol. Chem., 241, 800-806 (1966) ) ; Klebsiella pneumoniae, Serratia marcescens, Cryptococcus melibiosum [Biochim. Biophys. Acta.,293, 295-303 (1973)) ; and Bovine brain (Biochem. Biophys. Res. Comm.,68, 1133-1138 (1976)] ; Bacillus sp. No. 3 (product of Toyo Jozo Co.).

Among these, Aerobacter aerogenes, Klebsiella pneumoniaeand Serratia marcescens are known as etiologic microorganisms for pneumoniae and opportunistic infections (Standard Microbiology., p. 209-212, Igaku Shoin Publ., Tokyo) with inveterate infection resistant to chemotherapeutics and antibiotics. Culturing these microorganisms on an industrial scale is almost impossible. The Km-value of myo-inositol dehydrogenase produced by the yeast Cryptococcus melibiosum is approx. 11.0 mM for myo-inositol and approx. 0.07 mM for NAD, which are so high values of Km that it is not possible to get a sufficient reaction rate. (Enzyme Handbook, p. 6)

We have made a screening search to obtain microorganisms which can produce myo-inositol dehydrogenase, without non-infectious, higher activity, low Km-value on substrate myo-inositol and NAD, stabley and easily purify and isolated a microorganism designated Bacillus sp. No. 3 from a soil sample in a hot spring area, Atagawa, Higashi-Izu-cho, Kamo-gun,Shizuoka-ken, Japan.

Myo-inositol dehydrogenase produced by Bacillus sp. No. 3 has quite low Km-values for myo-inositol and NAD at pH 8.5 of approx. 0.6 mM and 0.04 mM, respectively, with high reactivity. Moreover the activity remaining after treating at 60 °C for 15 minutes in buffer solution is over approx. 95 %. The myo-inositol dehydrogeanse is a novel enzyme

which can act with a coenzyme of the NAD(P) group and the thio-NAD(P) group.

The novel enzyme myo-inositol dehydrogenase can also be produced by a myo-inositol dehydrogenase producing microorganism belonging to the genus Bacillus and isolated therefrom.

The myo-inositol producing microorganism belongs to the genus Bacillus, and Bacillus sp. No. 3, which has isolated by the present inventors is the most preferred microorganism.

The taxonomical properties of this strain are :

(a) Morphological properties:

Round edge with straight or slightly curved bacillus. Sizes are 0.5 ~ 0.7 x 1.5 ~ 3.5μm. Peritrical movement. Spore formations in an edge or subedge with sizes in 0.8 x 1.0 ~ 2.0 μm of elliptical to oval spores are observed. Microbial cells are swollen with spores. No polimorphism.

(b) Growth on various media:

Observations on various media, cultured for 1-2 days at 50-52°C, are as follows:

1. Nutrient agar plate medium : round and convex colonies. Smooth wettish surface with round edge. Ocherous or plate ocherous. No formation of soluble pigment.
2. Nutrient agar slant medium : good growth with filiform. Ocherous to plate ocherous. No formation of soluble pigment.
3. Liquid medium (aqueous peptone) : good growth with uniform turbidity.
4. Litmus milk medium : weakly acidic after 4-5 days.

GC mole % in DNA : 41.9 mole % (HPLC method)
Main isoprenoid quinone : MK - 7

(c) Physiological properties (+ = positive, (+)=weakly positive, = negative, NT= not tested)

| | | |
|---|---|---|
| Gram-strain | | + |
| KOH reaction | | − |
| Capsule formation | | − |
| Acid fastness stain | | − |
| OF-test (High Leifson) | | N T |
| OF-test (nitrogen source: $NH_4H_2PO_4$) | | F |
| Aeroboc growth | | + |
| Anaerobic growth | | + |
| Growth temperature | 70 ℃ | − |
| | 60 ℃ | + |
| | 37 ℃ | + |
| | 30 ℃ | − |
| Halotolerant NaCl conc. | % | |
| | 0 % | + |
| | 3 % | + |
| | 5 % | − |
| Growth pH | | |

| | | |
|---|---|---|
| | pH 5.6 | − |
| | pH 6.2 | + |
| | pH 9.0 | + |
| Gelatin hydrolysis | | − |
| Starch hydrolysis | | (+) |
| Casein hydrolysis | | − |
| Esculin hydrolysis | | + |
| Cellulose hydrolysis | | − |
| Tyrosine hydrolysis | | − |
| Arginine hydrolysis | | − |
| Catalase production | | + |
| Oxidase production | | + |
| Lecithinase production | | − |
| Urease production (SSR) | | − |
| Urease production (Chris) | | − |
| Indol production | | − |
| $H_2S$ production (detection: lead acetate paper) | | − |
| Acetoin production ($K_2HPO_4$) | | − |
| Acetoin production (NaCl) | | − |
| MR test | | − |
| Nitrate reduction | | |
| Gas detection | | − |
| $NO_2^-$ | | − |
| $NO_3^-$ | | + |

| | |
|---|---|
| Utilization of Simmons medium | |
| Citrate | − |
| Malate | − |
| Maleate | − |
| Malonate | − |
| Propionate | − |
| Gluconate | − |
| Succinate | − |
| Utilization of Christenssen medium | |
| Citrate | + |
| Malate | − |
| Maleate | − |
| Malonate | − |
| Propionate | + |
| Gluconate | − |
| Succinate | − |
| Gas production from glucose | − |
| Acid formation from sugar | |
| Adonitol | − |
| L (+) arabinose | − |
| Cellobiose | + |
| Dulsitol | − |
| Meso-erythritol | − |
| Fructose | + |
| Fucose | + |
| Galactose | + |

| | |
|---|---|
| Glucose | + |
| Glycerin | + |
| Inositol | + |
| Inulin | + |
| Lactose | + |
| Maltose | + |
| Mannitol | + |
| Mannose | + |
| Melezitose | − |
| Melibiose | + |
| Raffinose | − |
| Rhamnose | + |
| D-ribose | + |
| Salicin | + |
| L-sorbose | − |
| Sorbitol | − |
| Starch | + |
| Saccharose | + |
| Xylose | − |
| Trehalose | + |

According to the above taxonomical properties, the microorganism displays the specific characteristics of Gram positive bacillus, namely, it is 0.5 ~ 0.7 × 1.5 ~ 3.5µm in size, is peritrichal in movement, has spore formation with no polymorphism, promotes fermentative decomposition of glucose and acid formation, is catalase positive and oxidase positive, and is thermophilic and facultative anaerobic.

Among Gram-positive bacillus with specific properties of spore formation and aerobic growth, the strain belongs to be genus Bacillus.

According to Bergey's Mannual of Systematic Bacteriology, Vol. 2, there are illustrated the following 9 species of Bacillus with growth at high temperature (50 °C).

Bacillus acidocaldarius, B. subtilis, B. badius,
B. brevis, B. coagulans, B. licheniformis,
B. pantothenticus, B. schegelli and
B. stearothermophilus.

Among these, microorganisms growing in anaerobic conditions are B. coagulance and B. licheniformis.

The taxonomic properties of Bacillus in comparison with those of the present strain, according to Bergey's Mannual, are illustrated by comparing Bacillus coagulans (hereinafter designated C) and Bacillus licheniformis (hereinafter designated L), as follows:

+ = positive
(+) = weakly positive
- = negative
d = not identified as + or -
ND = no data

| | | C | L | The present strain |
|---|---|---|---|---|
| Oxidase production | | − | d | + |
| Swelling with spore | | d | − | + |
| Anaerobic growth | | + | + | + |
| Acetoin production | | + | + | − |
| Glucose (acid) | | + | + | + |
| L-arabinose (acid) | | + | + | + |
| Xyrose | | d | + | − |
| Mannitol | | d | + | + |
| Casein hydrolysis | | d | + | − |
| Gelatin hydrolysis | | d | + | − |
| Starch hydrolysis | | − | + | (+) |
| Citrate utilization | | + | + | − |
| Propionate utilization | | d | + | − |
| Tyrosine hydrolysis | | − | + | − |
| LV-reaction | | − | + | − |
| Indol production | | − | + | − |
| Halotolerant | 2 % | + | + | + |
| | 5 % | − | + | − |
| | 7 % | − | + | ± |
| | 10 % | − | ND | − |
| Growth tremperature | | | | |
| | 40 ℃ | + | + | + |
| | 50 ℃ | + | + | + |
| | 55 ℃ | + | + | + |
| | 60 ℃ | ND | ND | + |

| | | | |
|---|---|---|---|
| 70 ℃ | − | − | − |
| Nitrate reduction | d | + | − |
| GC mole % in DNA | 44.5 | 46.4 | 41.9 |
| | (Type) | (Type) | |
| | 44.3 | 42.9 | |
| | ~50.3 | ~49.9 | |

According to the above comparison, the present strain No. 3 has many identical properties with Bacillus coagulans but has specific differences as to acetoin production and GC mole % in DNA. Furthermore an observation on Litmus milk medium is different (not mentioned in the above comparison).

Accordingly, the present strain has been designated Bacillus sp. No. 3 and was deposited at the Fermentation Research Institute on 13 July 1990 and assigned the deposit No. FERM BP-3013.

For production of the enzyme, a myo-inositol dehydrogenase producing microorganism belonging to the genus Bacillus is cultured in a suitable medium.

A preferred example of the above myo-inositol dehydrogenase producing microorganism strain is Bacillus sp. No. 3. Since the taxonomical properties of bacterial are in general easily mutated, natural variants or artificial mutants produced by conventional artificial mutation treatment, for example by ultraviolet irradiation, radiation or a mutagen such as N-methyl-N-nitro-N-nitrosoguanidine and ethyl methansulfonate, are within the scope of the genus Bacillus and having myo-inositol dehydrogenase producing activity, and can be used in the present invention.

Culture of the above microorganism can be performed using the conventional culture conditions used for bacteria. In the present enzyme production, since myo-inositol dehydrogenase is an inducible enzyme by myo-inositol, culture is preferably carried out in a medium containing 0.5 ~ 5% myo-inositol to produce myo-inositol dehydrogenase with 10 ~ 300 times productivity.

Examples of components of the medium other than myo-inositol are a assimilable carbon source, a digestible nitrogen source and, if required inorganic salts.

Example of carbon source are glucose, fructose or saccharose, which are used in combination or alone. Examples of digestible nitrogen sources are peptone, meat extract or yeast extract, which are used in combination or alone. According to requirement, a phosphate, magnesium salt, calcium salt, potassium salt, sodium salt or various heavy metal salts such as a ferric salts or manganese salt can he added. Other known assimilable carbon sources and digestible nitrogen salts can also be used.

Culture is generally by shake culture or aeration culture with agitation. For industrial production submerged aeration culture with agitation is preferred. The culture temperature can be altered within a range for production of the enzyme, and is generally 40 ~ 60 °C, preferably approx. 50°C.

The culture time depends on the conditions of culture, and can be set up for maximum production of the enzyme. It is generally 1 ~ 2 days.

These composition of the medium, medium condition, culture temperature, agitation speed and air flow should naturally be adjusted to preferred settings. An anti-foaming agent such as silicon oil, or vegitable oil can also be used if required.

Since the thus produced myo-inositol dehydrogenase exists as an end enzyme in bacterial cells, the cultured cells are collected by means of filtration or centrifugation, and the bacterial cells are decomposed by mechanical destruction such as by ultrasonication, French press treatment, glass beads treatment, freezing-thawing, or enzymatic digestion such as lysozyme treatment, to obtain a crude extract.

Further purified myo-inositol dehydrogenase can be obtained by known conventional purification methods of isolation of a protein or enzyme. For example, the enzyme can be precipitated by a salting-out method by adding ammonium sulfate or sodium salfate to the crude extract. The precipitated enzyme can further be purified by a combination of chromatography using a molecular sieve and a resin, electrophoresis or ultra-centrifugation.

The purification can be achieved by considering the nature of myo-inositol dehydrogenase. For example, the above precipitae dissolved in water or buffer solution is dialysed, if required, by a semi-permeable membrane, and treated by chromatography using an ion-exchange resin such as DEAE-cellulose, DEAE-Sephacel, DEAE-Sepharose, DEAE-Sephadex, Q-Sepharose (Pharmacia Corp., trade name), DEAE-Toyoperal (Toso Corp.) or hydroxy apatite, a hydrophobic chromatographic resin such as octyl-Sepharose or phenyl-Sepharose (Pharmacia Corp.) or another affinity chromatographic resin. Further molecular sieve chromatography using a gel-filtration agent such as Sephadex G-100 or Sephacryl S-200 can be applied. If required desalting by means of a semi-premeable membrane can also be applied.

A purified myo-inositol dehydrogenase powder preparation can be prepared by lyophilization with addition of 0.05 ~ 10 % of a stabilizing agent, for example sugars such as mannitol, saccharose or sorbitol, amino acids such as glutamate or glycine, or a peptide such as bovine albumin.

The myo-inositol dehydrogenase has the following properties;

1. Substrate specificity:

| | |
|---|---|
| myo-inositol | 100 % |
| glucose | 0 |
| fructose | 0 |
| galactose | 0 |
| sorbitol | 0 |
| mannose | 0 |
| maltose | 0 |
| saccharose | 0 |
| lactose | 0 |

2. Enzyme action:

The enzyme catalyzes essentially a reaction of myo-inositol and NAD to generate myo-inosose and reduced NADH, as shown below:

$$\text{myo-inositol} + \text{NAD} \rightleftharpoons \text{myo-inosose}^{*} + \text{reduced NADH}$$

*(2, 4, 6/3, 5-pentahydroxy cyclohexanone)

3. Molecular weight:

130,000± 15,000

Measured by TSK-gel G 3000 SW 8 (Toso Co., 0.75 × 60 cm).

Elution : 0.1 M phosphate buffer (pH 7.0) containing 0.2 M Nacl.

Standard : the following molecular markers (Oriental yeast Co) are used:

| | |
|---|---|
| M. W. 12,400 | Cytochrome C |
| M. W. 32,000 | adenylate kinase |
| M. W. 67,000 | bovine albumin |
| M. W. 142,000 | lactate dehydrogenase |
| M. W. 290,000 | glutamate dehydrogenase |

4.- Isoelectric point:

pH 4.5 ± 0.5

Measured by elelctrofocussing using carrier ampholite at 4°C, 700 V, for 40 hours. The activity of a fraction of each enzyme is measured.

5. Km-value: 0.64 mM (myo-inositol), 0.004 mM (NAD)

The Km-value for myo-inositol is measured in various concentarations of myo-inositol in a reaction mixture of:

100 mM Tris-HCl buffer (pH 8.5)
5 U diaphorase (Toyo Jozo Co.)
1 mM NAD (Oriental yeast Co.)
0.025 % NBT (Wako Pure Chem. Co.)

In the reaction mixture, NAD is replaced by 15 mM myo-inositol, and the concentration of NAD is varied to measure the Km-value for NAD.

The results are as shown above.

Furthermore in the reaction mixture 1 mM NAD is replaced by 1 mM thio-NAD (Sigma Co.), and the Km-value for myo-inositol is measured. The result is as shown below:

Km-value: 10.0 mM (myo-inositol)

In the reaction mixture, 1 mM thio-NAD is replaced by 150 mM myo-inositol, and the Km-value for thio-NAD is measured.

Km-value : 0.17 mM (thio-NAD)

The Km-value in the reaction of NADP and myo-inositol are measured. The results are as shown below:

Km-value : 0.19 mM (NADP)

Km-value : 30.91mM (myo-inositol)

The Km-values in the reaction of the thio-NADP and myo-inositol are as shown below:

Km-value : 2.54 mM (thio-NADP)

Km-value : 179.62 mM (myo-inositol)

As clearly known from above, the enzyme can be reacted using NAD(P) and thio-NAD(P) as the coenzymes.

6. Optimum pH:

In an assay method for enzyme activity as illustrated hereinafter, 100 mM Tris-HCl buffer (pH 8.5) in the reaction mixture is replaced by 100 mM phosphate buffer (pH 6.5-8.0, -○-), 100 mM Tris-HCl buffer (pH 8.9-9.0, -□ -) and 100 mM glycine-NaOH buffer (pH 9.0-10.0,- ■-), and incubated. The result is shown in Fig. 4.

A maximum activity is observed at approx. pH 9.5.

7. pH-stability:

The residual activity of the enzyme (1 U/ml, 40 mM buffer solution) is measured in various buffer solutions, i. e. acetate buffer (pH 4.5-6.0, - ▲-) ; phosphate buffer (pH 6.0-8.0, -○-) ; Tris-HCl buffer (pH 8.0-9.5, - □-) and glycine-NaOH buffer (pH 9.0-10, -■-) after heating at 50°C for 15 mins. The enzyme is stable at pH 6.5-9.0 with a residual activity of over 80 %, as shown in Fig.3.

8. heat-stability:

The enzyme, dissolved in 20 mM Tris-HCl buffer (pH 7.0) to produce a 1 U/ml solution, is incubated for 15 min. at various temperatures, and the residual activity is measured.

The results are as shown in Fig. 1. The enzyme is stable up to 60°C while maintaining a residual activity of over 95 %.

9. Optimum temperature:

The enzyme activity is measured at 35, 40, 50, 55, 60 and 65°C, respectively, in 100 mM Tris-HCl buffer (pH 8.5) according to the assay method illustarted hereinafter. The reaction was stopped in each case after 10 mins. incubation by adding 0.1 N HCl (2 ml), whereupon the optical absorption was measured at 550nm. The enzyme shows maximum activity at 60 °C as shown in Fig. 2.

10. Assay method of myo-inositol dehydrogenase

(1) Reaction mixture:

| | |
|---|---|
| 100 mM | Tris-HCl buffer (pH 8.5) |
| 15 mM | Myi-inositol (Wako Pure Chem. Co) |
| 1 mM | NAD (Oriental Yeast Co.) |
| 5 U | Diaphorase (Toyo Jozo Co.) |
| 0.025 % | NBT (Wako Pure Chem. Co.) |

(2) Enzyme Assay:

The above reaction mixture (1 ml) is incubated in a small test tube at 37°C for 5 mins. Diluted enzyme solution

(0.02 ml) is added and stirred to initiate the reaction. After exactly 10 mins., 0.1 N HCl (2.0 ml) is added and the mixture stirred to stop the reaction. Absorption at 550 nm ($A_{550}$ nm) is measured to obtain absorption $A_1$. The assay is repeated using the above reaction mixture, except that myo-inositol was not included. The mixture is also treated in the same manner as described above and its absorption Ao is measured.

(3) Calculation of enzyme activity:

$$U/ml=\frac{(A_1\text{-}A_0)}{18.3}\times\frac{1}{10}\times\frac{3.02}{0.02}\times Z$$

wherein

18.3 : molecular absorption coefficient ($cm^2/\mu mol$)

Z : dilution factor

In the enzymatic reaction hereinbefore illustrated, $A_1$ or $B_2$ is a coenzyme of the thio-NADP-group, thio-NAD group, NADP group or NAD group. Examples of the thio-NADP group or thio-NAD group are thionicotinamide adenine dinucleotide phosphate (thio-NAD) and thionicotinamide hypoxanthine dinucleotide phosphate or thionicotinamide adenine dinucleotide (thio-NAD) and thionicotinamide hypoxanthine dinucleotide. Examples of the NADP group or NAD group are nicotinamide adenine dinucleotide phosphate (NADP), acetylpyridine adenine dinucleotide phosphate (acetyl NADP) or nicotinamide hypoxanthine dinucleotide phosphate (deamino NADP) and nicotinamide adenine dinucleotide (NAD), acetylpyridine adenine dinucleotide (acetyl NAD) and nicotinamide hypoxanthine dinucleotide (deamino NAD).

In the present invention, when $A_1$ is of the thio-NAD(P) group, $B_1$ is of the NAD(P)H group, and when $A_1$ is of the NAD(P) group, $B_1$ is of the thio-NAD(P)H group. Hence at least one will be a thio-type coenzyme.

Furthermore when the coenzymes are of the thio-NAD group and NAD group, any of the thio-NAD group and NAD group hereinabove illustrated can be selected. When the coenzymes are of the thio-NAD(P) group and NAD(P) group, any of the thio-NAD group or thio-NADP group and NAD group or NADP group can be selected.

In a composition for an assay of myo-inositol according to the present invention, the concentration of $A_1$ and $B_1$ is generally 0.02-100 mM, preferably 0.05-20 mM, and the concentration of myo-inositol dehydrogenase is generally 5-1000 U/ml, preferably 2-500 U/ml. Amounts exceeding the above ranges are acceptable.

$A_1$ and $B_1$ are used in excess as compared with myo-inositol and are in excess as compared with the Km-value (mM) of myo-inositol dehydrogenase for $A_1$ and $B_1$. Specifically a 20-10,000 times molar excess relative to myo-inositol is preferred.

In the present invention, a fourth component of dehydrogenase X which does not act on myo-inositol and which constitutes a reaction from $B_2$ to $B_1$, and a substrate for the said dehydrogenase X, may be combined to effect a cycling reaction of the formula (II) in which a reaction for regenerating the $B_1$ is added to the reaction $B_1 \rightarrow B_2$, and the amount of $A_2$ generated is measured.

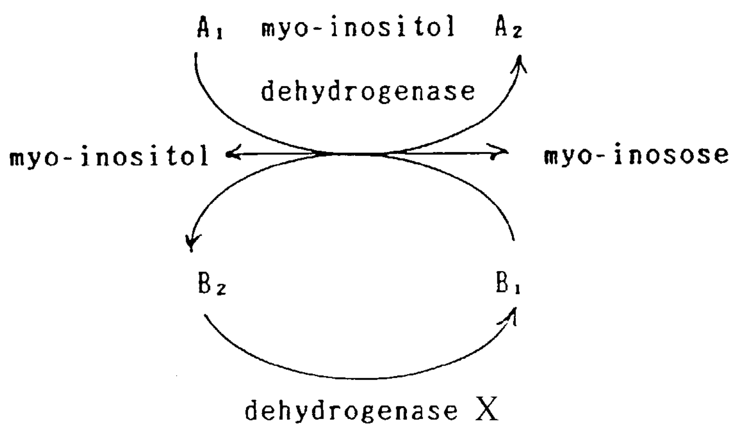


(II)

wherein $A_1$ is a thio-NADP group, thio-NAD group NADP group or NAD group compound, $A_2$ is a reduced form of $A_1$, when $A_1$ is a thio-NADP group or thio-NAD group compound, $B_1$ is a reduced NADP group or reduced NAD group compound, and when $A_1$ is a NADP group or NAD group compound, $B_1$ is a reduced thio-NADP group or reduced thio-NAD group compound, and wherein $B_2$ is an oxidized form of $B_1$. The reaction from $B_2$ to $B_1$ is an enzymatic reaction which regenerates $B_1$ by an action of the dehydrogenase X with coenzyme of $B_2$. Thus the dehydrogenase X is added supplementary for regenerating the $B_1$, and therefore the amount of $B_1$ can be reduced. $B_1$ can be replaced by $B_2$ or a mixture of $B_1$ and $B_2$. In this case the amount of $B_1$ or/and $B_2$ is not limited, but is generally below 1/10 mole of $A_1$, preferably 1/50 ~ 1/1000 mole, or less.

In a composition for an assay of myo-inositol using component (4) according to the present invention, the concentration of $A_1$ is generally 0.02 ~ 100 mM, preferably 0.05 ~ 20 mM, the concentration of $B_2$ or/and $B_1$ is generally 0.05 ~ 5000 μM, preferably 5 ~ 500 μM, and the concentration of myo-inositol dehydrogenase is generally 5 ~ 1000 U/ml, preferably 20 ~ 500 U/ml. The concentration of the dehydrogenase X is 20 times (U/ml) or more as compared with the Km-value of the second myo-inositol dehydrogenase for $B_2$, for example preferably 1 ~ 100 U/ml. The substrate for the dehydrogenase X is used in excess, preferably 0.05 ~ 20 mM or more.

Examples of the dehydrogenase X and a substrate for the dehydrogenase X are as follows.

$B_2$: NAD group or thio-NAD group;

Alcohol dehydrogenase (EC.1.1.1.1) and ethanol,

Glycerol dehydrogenase (EC.1.1.1.6)(E. coli) and glycerol,

Glycerol-3-phosphate dehydrogenase (EC.1.1.1.8) (rabbit muscle) and L-glycerol-3-phosphate,

Maleic dehydrogenase (EC.1.1.1.37)(porcine heart muscle, bovine heart muscle) and L-malate, and

Glyceraldehyde phosphate dehydrogenase (EC.1.2.1.12) (rabbit muscle, liver, yeast, E. coli) and D-glyceraldehyde phosphate and phosphate.

$B_2$ : NADP group or thio-NADP group;

Glucose-6-phosphate dehydrogenase (EC.1.1.1.49) (yeast) and glucose-6-phosphate,

Isocitrate dehydrogenase (EC.1.1.1.42)(yeast, porcine heart muscle) and isocitrate,

Glyoxylate dehydrogenase (EC.1.2.1.17)(Pseudomonas oxalaticus) and CoA and glyoxylate,

Phosphogluconate dehydrogenase (EC.1.1.1.44)(rat liver, beer yeast, E. coli) and 6-phosphogluconate,

Glyceraldehyde dehydrogenase (EC.1.2.1.13) (chlorophyll) and D-glyceraldehyde-3-phosphate and phosphate, and

Benzaldehyde dehydrogenase (EC.1.2.1.7) (Pseudomonas fluorescens) and benzaldehyde.

In the present invention, a further 5th component of a dehydrogenase Y which does not act on myo-inositol and which constitutes a reaction from $A_2$ to $A_1$, and a substrate for the said dehydrogenase Y may be combined to effect a cycling reaction of the formula (III) in which a reaction for regenerating the $A_1$ is added to the reaction $A_1 \rightarrow A_2$, and the amount of $B_1$ decreased is measured.

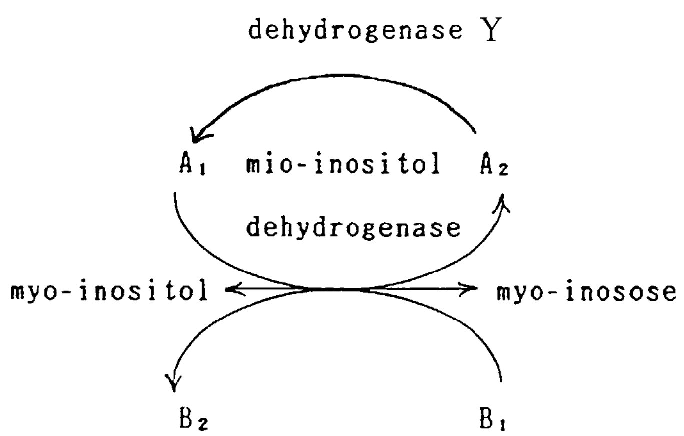


(III)

wherein $A_1$ is a thio-NADP group, thio-NAD group, NADP group or NAD group compound, $A_2$ is a reduced form of $A_1$, when $A_1$ is a thio-NADP group or thio-NAD group compound $B_1$ is a reduced NADP group or reduced NAD group compound, and when $A_1$ is a NADP group or NAD group compound $B_1$ is a reduced thio-NADP group or reduced thio-NAD group compound, and wherein $B_2$ is an oxidized form of $B_1$ . The reaction from $A_2$ to $A_1$ is an enzymatic reaction which regenerates $A_1$ by an action of the dehydrogenase Y with a coenzyme of $A_2$. Thus the dehydrogenase Y is added supplementary for regenerating the $A_1$, and therefore the amount of $A_1$ can be reduced. $A_1$ can be replaced by $A_2$ or a mixture of $A_1$ and $A_2$. In this case the amount of $A_1$ or/and $A_2$ is not limited, but is generally below 1/10 mole of $B_1$, preferably 1/50 ~1/1000 mole, or less.

In a composition for an assay of myo-inositol using component (5) according to the present invention, the concentration of $B_1$ is generally 0.02 ~ 100 mM, preferably 0.05 ~ 20 mM, the concentration of $A_2$ or/and $A_1$ is generally 0.05 ~ 5000 μM, preferably 5 ~ 500 μM, and the concentration of myo-inositol dehydrogenase is generally 5 ~ 1000 U/ml, preferably 20 ~ 500 U/ml. The concentration of the dehydrogenase Y is 20 times (U/ml) or more as compared with the Km-value of the third myo-inositol dehydrogenase for $A_2$, for example preferably 1 ~ 100 U/ml. The substrate for the dehydrogenase Y is used in excess, preferably 0.05 ~ 20 mM or more.

Examples of the dehydrogenase Y and the substrate for the dehydrogenase Y are as follows:

$A_1$: NAD group or thio-NAD group;
Alcohol dehydrogenase (EC.1.1.1.1) and acetoaldehyde,
Glycerol dehydrogenase (EC.1.1.1.6) (E.coli) and dihydroxyacetone
Glycerol-3-phosphate dehydrogenase (rabbit muscle) and dihydroxyacetone phosphate,
Maleic dehydrogenase (EC.1.1.1.37)(porcine heart muscle, bovine heart muscle) and oxaloacetate and
Glyceraldehyde phosphate dehydrogenase (EC.1.2.1.12) (rabbit muscle, liver, yeast, E. coli) and 1, 3 -diphospho-D-glycerate.
$A_1$ : NADP group or thio-NADP group;
Glucose-6-phosphate dehydrogenase (EC.1.1.1.49) (yeast) and gluconolactone-6-phosphate, and
Glyceraldehyde phosphate dehydrogenase (EC.1.2.1.13) (chlorophyll) and 1, 3-diphospho-D-glycerate.

The myo-inositol dehydrogenase used in the composition for assay of myo-inositol according to the present invention can be an enzyme having reactivity on a substrate of myo-inositol together with a suitable coenzyme of the NAD group, preferably of the NAD or thio-NAD group, or preferably of the thio-NAD and NADP group, or preferably of the NADP or thio-NADP group, preferably thio-NADP. Its suitability can be confirmed by using the said coenzyme and substrate.

In the reaction medium composition two coenzymes are selected by considering their relative activity with myo-inositol dehydrogenase. Thereafter the pH condition, considering the optimum pH of the forward reaction and the reverse reaction, is adjusted such that the ratio of the forward reaction rate to the reverse reaction rate approaches 1:1.

Myo-inositol dehydrogenase produced by Bacillus sp. No. 3 (product of Toyo Jozo Co.) has a relative activity of approx. 10 ~ 15 % when the coenzyme thio-NAD is used, as compared to use of NAD. The optimum pH is approx. 9.5 for the forward reaction and approx. 7 ~ 7.5 for the reverse reaction. The enzyme can utilize coenzymes of both the NAD group and the NADP group.

In the present invention, myo-inositol dehydrogenase from a single origin or a plurality of origins can be used.

Myo-inositol in a specimen can be assayed by adding 0.001 ~ 0.5 ml of the specimen to the assay composition containing the above components (1) - (3), components (1) - (4) or components (1) - (3) and (5), carrying out a reaction at approx. 37 °C, and then measuring the amount of generated $A_2$ or consumed $B_1$ over an interval spanning two time points after starting the reaction. For example the measurement can be carried out for a minute between 3 mins. and 4 mins. after starting, or for five minutes between 3 mins. and 8 mins. after starting the reaction. The measurement is effected by determining the changes of absorption at each optical absorption. For example, when $A_2$ is thio-NADH and $B_1$ is NADH, the generated $A_2$ is measured by an increase of absorption at 400 nm or consumed $B_1$ is measured by a decrease of absorption at 340 nm and the thus-obtained value is compared with the value of a known concentration of reference myo- inositol. Thus the concentration of myo-inositol in a specimen can be measured in real time.

According to the assay method of the present invention, since myo-inositol itself existing in a specimen is introduced into the enzymatic cycling reaction, any coexisting substances in the specimen do not effect the result. Hence a control measurement of a specimen not containing myo-inositol is not required. Thus a simple assay system using a rate assay can be achieved.

Measuring a value of $A_2$ or $B_1$ can be performed not only by absorbancy, but also by other known analytical methods instead.

As explained above, the present invention has advantages in that no measurement error can occur, due to the use of coenzymes each having a different absorption in its reduced form. The amounts of myo-inositol can also be assayed precisely and rapidly even with a small amount of specimen, due to combining enzymatic cycling reactions. In the present invention, use of heat stable myo-inositol dehydrogenase having a remaining activity of over 95 % at 65°C is preferable.

## Examples

The following Examples further illustrate the present invention.

Example 1

Reagents:

| | |
|---|---|
| 40 mM | Glycine-NaOH buffer (pH 10.0) |
| 2 mM | Thio-NAD (Sankyo Co.) |
| 0.2 mM | reduced NAD (Oriental Yeast Co.) |
| 150 U/ml | Myo-inositol dehydrogenase (Toyo Jozo Co. Bacillus sp. No. 3) |

Procedure:

The above reagent mixture (1 ml) was put into cuvettes and 20 µl of each of a range of concentrations of myo-inositol solution (0, 10, 20, 30, 40 and 50 µM, respectively) was added thereto, with a reaction temperature of 37°C. After incubation commenced, the difference in absorbance at 400 nm at 2 mins. and 7 mins. was measured. The results are shown in Fig. 5, from which it can be seen that a linear relation between the amount of myo-inositol and the change in absorption was observed.

Example 2

Reagents:

| | |
|---|---|
| 40 mM | Glycine-NaOH buffer (pH 9.5) |
| 2 mM | Thio-NAD (Sankyo Co.) |
| 0.1 mM | reduced deamino NAD (Sigma Co.) |
| 200 U/ml | Myo-inositol dehydrogenase (Toyo Jozo Co. Bacillus sp. No. 3) |

Procedure:

The above reagent mixture (1 ml) was put into a cuvettes and 50 µl of each of a range of concentrations of myo-inositol solution (0, 2, 4, 6, 8 and 10 µM, respectively) was added thereto. The mixture was incubated at 37 °C for 60 minutes. The reaction was then stopped by adding 0.5 % sodium dodecyl sulfate (1 ml). Absorbance at 400 nm was measured. The results are shown in Fig. 6, from which can be seen a good linear quantitative curve.

Example 3

Reagents:

| | |
|---|---|
| 50 mM | Glycine-NaOH buffer (pH 10.0) |
| 0.2 mM | reduced NAD (Oriental yeast Co.) |
| 4 mM | thio-NAD (Sankyo Co.) |
| 250 U/ml | Myo-inositol dehydrogenase (Toyo Jozo Co. Bacillus sp. No. 3) |
| 0.2 % | Triton X - 200 |

Procedure:

The above reagent mixture (1 ml) was put into cuvettes and 20 µl of each of three different sera was added thereto, with the reaction temperature at 37 °C . After incubation commenced, the difference in absorbance at 400 nm at 5 min. and 6 min. was measured.

50 µM myo-inostiol solution (standard solution) and distilled water (reagent blank) were treated in the same manner as above, and the amount of myo-inositol in serum samples was calculated from the difference in absorbance between the standard solution and the samples.

In the table hereinbelow, the results obtained from three different sera are shown.

| | Difference Absobance (mAbs) | Myo-inositol concentration |
|---|---|---|
| Reagent blank | 2 | - |
| Standard solution | 29 | 50 µM |
| Serum 1 | 25 | 42.6 µM |
| Serum 2 | 21 | 35.2 µM |
| Serum 3 | 31 | 53.7 µM |

Example A

Reagents:

| | |
|---|---|
| 40 mM | Glycine-NaOH buffer (pH 10.0) |
| 15 mM | NADP (Oriental yeast Co.) |
| 50 µM | thio-NAD (Sankyo Co.) |
| 0.4 M | Ethanol |
| 30 U/ml | Alcohol dehydrogenase (Oriental yeast Co.) |
| 250 U/ml | Myo-inositol dehydrogenase (Toyo Jozo Co. Bacillus sp. No. 3) |

Procedure:

The above reagent mixture (1 ml) was put into cuvettes and 50 µl of each of concentrations of myo-inositol solution (0, 20, 40, 60, 80 and 100 µM, respectively) was added thereto, with a reaction temperature of 37 °C . After incubaion commenced, the difference in absorbancy at 340 nm at 3 mins and 8 mins. was measured. The results are shown in Fig. 7.

Example 5

Reagents:

| | |
|---|---|
| 50 mM | Phosphate buffer (pH 7.0) |
| 0.25 mM | reduced NADP (Oriental yeast Co.) |
| 50 µM | thio-NAD (Sankyo Co.) |
| 5 mM | Dihydroxyacetone phosphate |
| 10 U/ml | Glycerol-3-phosphate dehydrogenase (Boehringer Mannheim, rabbit muscle) |
| 250 U/ml | Myo-inositol dehydrogenase (Toyo Jozo Co. Bacillus sp. No. 3) |

Procedure:

The above reagent mixture (1 ml) was put into cuvettes and 50 µl of each of concentrations of myo-inositol solution (0, 50, 100, 150, 200 and 250 µM, respectively) was added thereto, with a reaction temperature of 37°C. After incubaion

commenced, the difference in absorbance at 340 nm at 3 mins. and 8 mins. was measured. The results are shown in Fig. 8.

Example 6

| | |
|---|---|
| Culture Bacillus sp. No. 3: | |
| Yeast extract (Kyokuto Seiyaku Co.) | 2 % |
| Peptone (Kyokuto Seiyaku Co.) | 2 % |
| $K_2HPO_4$ (Wako Pure Chem. Co.) | 0.2 % |
| $CaCl_2$ (Wako Pure Chem. Co.) | 0.02 % |
| $MgSO_4 \cdot 7H_2O$ (Wako Pure Chem. Co.) | 0.05 % |
| Myo-inositol (Wako Pure Chem. Co.) | 2 % |
| pH 7.3 | |

100 ml of a liquid medium comprising the above composition was sterilized in a 500 ml Erlenmeyer flask at 120 °C for 20 mins. One loopful of Bacillus sp. No. 3 was inoculated into the medium and the medium was cultured at 50 °C with stirring at 120 r.p.m. for 30 hours to obtain a cultured mass (85 ml)(enzyme activity: 1.2 U/ml). 20 1 of a liquid medium comprising the above composition with 0.1% disform CB 442 (Nihon Yushi Co.) added was sterilized in a 30 1 jar fermenter by heating. 85 ml of the pre-cultured seed culture obtained in the step above was inoculated therein and the mixture was cultured at 50°C, with an aeration of 20 l/ml., inner pressure of 0.4 kg/cm$^2$, and agitation of 150 r.p.m. for 24 hours to obtain a cultured mass (18.0 l)(enzyme activity: 1.8 U/ml).

Example 7

Purification of enzyme

Bacterial cells collected by centrifugation from the cultured broth obtained in Example 6 were suspended in 20 mM phosphate buffer (pH 7.5, 5 1) containing 0.1% lysozyme (Eizai Co.) and solubilized at 37°C for 1 hour; then the mixture was centrifuged to remove precipitate and to obtain a supernatant solution (4500 ml) (activity: 6 U/ml). Acetone (1.8 1) was added to the supernatant solution to separate the precipitate, which was dissolved in 20 mM phosphate buffer to obtain a crude extract (1 lit., 24.2 U/ml).

Ammonium sulfate (200 g) was added to the solution, which was mixed well by stirring and then centrifuged to separate the precipitate. An additional 250 g of ammonium sulfate was then added to the supernatant solution, and the solution was centrifuged to obtain a new precipitate. The new precipitate was dissolved in 20 mM phosphate buffer (pH 7.5) to obtain an enzyme solution (500 ml, specific activity 36.3 U/ml), and the resultant solution was dialyzed overnight against 20 mM phosphate buffer (pH 7.5, 20 lit.). The dialyzed enzyme solution was charged on a column of DEAE-Sepharose CL-6B (Pharmacia Co.) (250 ml), which was buffered with 20 mM phosphate buffer (pH 7.5), washed with 20 mM phosphate buffer containing 0.1 M KCl, (pH 7.5, 1 lit) and eluted with 20 mM phosphate buffer containing 0.3 M KCl (pH 7.5) to obtain an enzyme solution (350 ml, activity 35.2 U/ml). The enzyme solution was dialyzed overnight against 10 mM phosphate buffer (pH 7.0, 20 lit.). Bovine serum albumin (Sigma Co., 0.2 g) was dissolved in the thus-obtained enzyme solution, then the solution was lyophilized to obtain the lyophilized enzyme (1.1 g, 10.6 U/mg).

**Claims**

**1.** A method of assaying a specimen for myo-inositol which comprises reacting the specimen with:

a) myo-inositol dehydrogenase using one of the thionictoinamide adenine dinucleotide phosphate group (thio-NADP group) or thionicotinamide adenine dinucleotide group (thio-NAD group) and one of the nicotinamide adenine dinucleotide phosphate group (NADP group) or nicotinamide adenine dinucleotide group (NAD group) as coenzymes, which myo-inositol dehydrogenase catalyzes a reversible reaction forming myo-inosose from myo-inositol,

b) $A_1$, and

c) $B_1$;

to effect the cycling reaction:

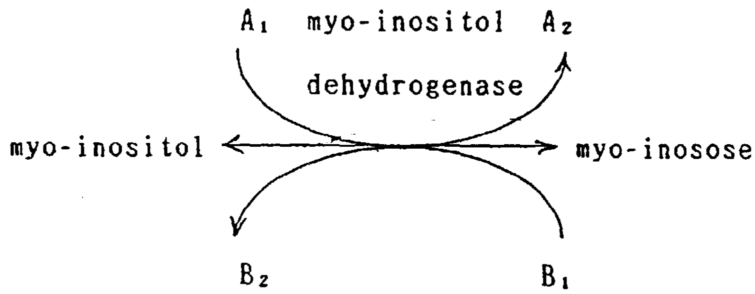


wherein:

$A_1$ is a thio-NADP, thio-NAD, NADP or NAD group compound,
$A_2$ is a reduced form of $A_1$,
$B_1$ is a reduced NADP or reduced NAD group compound when $A_1$ is a thio-NADP or thio-NAD group compound or $B_1$ is a reduced thio-NADP or reduced thio-NAD group compound when $A_1$ is a NADP or NAD group compound, and
$B_2$ is an oxidized form of $B_1$; and
measuring the amount of $A_2$ or $B_1$ generated or consumed in the cycling reaction.

**2.** A method of assaying a specimen for myo-inositol which comprises reacting the specimen with:

a) myo-inositol dehydrogenase using one of the thio-NADP group or thio-NAD group and one of the NADP group or NAD group as coenzymes, which myo-inositol dehydrogenase catalyzes a reversible reaction forming myo-inosose from myo-inositol,
b) $A_1$,
c) $B_1$ or/and $B_2$, and
d) a dehydrogenase (dehydrogenase X) which does not act on myo-inositol and which catalyses a reaction forming $B_1$ from $B_2$, and a substrate for the said dehydrogenase X;

to effect the cycling reaction:

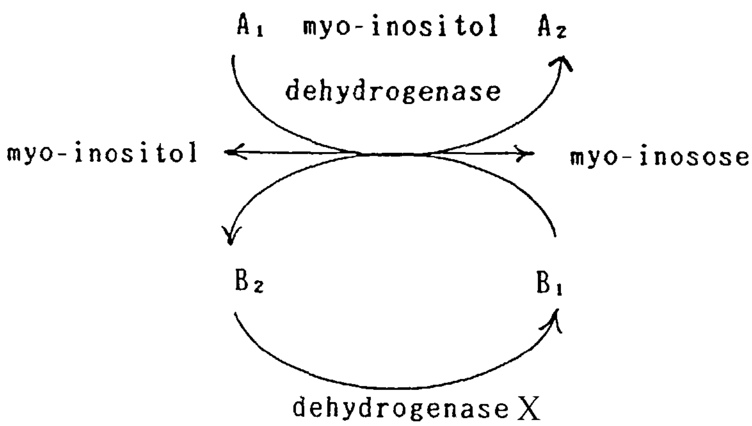


wherein:

$A_1$ is a thio-NADP, thio-NAD, NADP or NAD group compound,
$A_2$ is a reduced form of $A_1$,
$B_1$ is a reduced NADP or reduced NAD group compound when $A_1$ is a thio-NADP or thio-NAD group compound or $B_1$ is a reduced thio-NADP or reduced thio-NAD group compound when $A_1$ is a NADP or NAD group compound, and
$B_2$ is an oxidized form of $B_1$;
the reaction from $B_2$ to $B_1$ being an enzymatic reaction which generates $B_1$ from $B_2$ by an action of the dehydrogenase X with a coenzyme; and

measuring the amount of $A_2$ generated in the cycling reaction.

**3.** A method of assaying a specimen for myo-inositol which comprises reacting the specimen with:

a) myo-inositol dehydrogenase using one of the thio-NADP group or thio-NAD group and one of the NADP group or NAD group as coenzymes, which myo-inositol dehydrogenase catalyzes a reversible reaction forming myo-inosose from myo-inositol,
b) $A_1$ or/and $A_2$,
c) $B_1$ and
d) a dehydrogenase (dehydrogenase Y) which does not act on myo-inositol and which catalyses a reaction from $A_2$ to $A_1$, and a substrate for the said dehydrogenase Y;

to effect the cycling reaction:

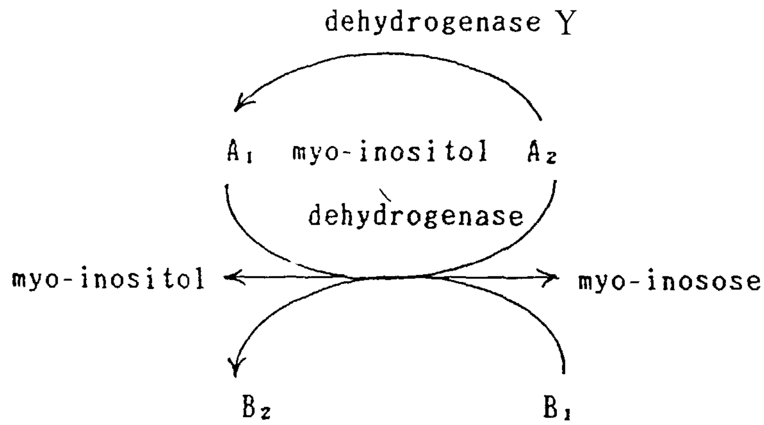


wherein:

$A_1$ is a thio-NADP, thio-NAD, NADP or NAD group compound,
$A_2$ is a reduced form of $A_1$,
$B_1$ is a reduced NADP or reduced NAD group compound when $A_1$ is a NADP or NAD group compound, and
$B_2$ is an oxidized form of $B_1$;
the reaction from $A_2$ to $A_1$ being an enzymatic reaction which generates $A_1$ from $A_2$ by an action of the dehydrogenase Y with a coenzyme; and
measuring the amount of $B_1$ consumed in the cycling reaction.

**4.** A method according to any one of the preceding claims wherein the thio-NADP group is thionicotinamide adenine dinucleotide phosphate (thio-NADP) or thionicotinamide hypoxanthine dinucleotide phosphate.

**5.** A method according to any one of claims 1 to 3 wherein the thio-NAD group is thionicotinamide adenine dinucleotide (thio-NAD) or thionicotinamide hypoxanthine dinucleotide.

**6.** A method according to any one of the preceding claims wherein the NADP group is nicotinamide adenine dinucleotide phosphate (NADP), acetylpyridine adenine dinucleotide phosphate (acetyl NADP) or nicotinamide hypoxanthine dinucleotide phosphate (deamino-NADP).

**7.** A method according to any one of claims 1 to 5 wherein the NAD group is nicotinamide adenine dinucleotide (NAD), acetylpyridine adenine dinucleotide phosphate (acetyl NAD), or nicotinamide hypoxanthine dinucleotide phosphate (deamino-NAD).

**8.** A reagent composition suitable for assaying myo-inositol which comprises:

a) myo-inositol dehydrogenase using one of the thionicotinamide adenine dinucleotide phosphate (thio-NADP group) or thionicotinamide adenine dinucleotide group (thio-NAD group) and one of the nicotinamide adenine dinucleotide phosphate group (NADP group) or nicotinamide adenine dinucleotide group (NAD group) as coenzymes, which myo-inositol dehydrogenase catalyzes a reversible reaction forming myo-inosose from myo-inositol,

b) $A_1$ and
c) $B_1$;

wherein:

$A_1$ is a thio-NADP, thio-NAD, NADP or NAD group compound,
$B_1$ is a reduced NADP or reduced NAD group compound when $A_1$ is a thio-NADP or thio-NAD compound or $B_1$ is a reduced thio-NADP or reduced thio-NAD group compound when $A_1$ is a NADP or NAD group compound.

**9.** A reagent composition suitable for assaying myo-inositol which comprises:

a) myo-inositol dehydrogenase using one of the thio-NADP group or thio-NAD group and one of the NADP group or NAD group as coenzymes, which myo-inositol dehydrogenase catalyzes a reversible reaction forming myo-inosose from myo-inositol,
b) $A_1$,
c) $B_1$ or/and $B_2$, and
d) a dehydrogenase (dehydrogenase X) which does not act on myo-inositol and which catalyzes a reaction forming $B_1$ from $B_2$, and a substrate for the said dehydrogenase X,

wherein:

$A_1$ is a thio-NADP, thio-NAD, NADP or NAD group compound,
$B_1$ is a reduced NADP or reduced NAD group compound when $A_1$ is a thio-NADP or thio-NAD group compound or $B_1$ is a reduced thio-NADP or reduced thio-NAD group compound when $A_1$ is a NADP or NAD group compound, and
$B_2$ is an oxidized form of $B_1$.

**10.** A reagent composition suitable for assaying myo-inositol which comprises:

a) myo-inositol dehydrogenase using one of the thio-NADP group or thio-NAD group and one of the NADP group or NAD group as coenzymes, which myo-inositol dehydrogenase catalyzes a reversible reaction forming myo-inosose from myo-inositol,
b) $A_1$ or/and $A_2$
c) B1 and
d) a dehydrogenase (dehydrogenase Y) which does not act on myo-inositol and which catalyzes a reaction from $A_2$ to $A_1$, and a substrate for the said dehydrogenase Y;

wherein:

$A_1$ is thio-NADP, thio-NAD, NADP or NAD group compound,
$B_1$ is a reduced NADP or reduced NAD group compound when $A_1$ is a thio-NADP or thio-NAD group compound or $B_1$ is a reduced thio-NADP or reduced thio-NAD group compound when $A_1$ is a NADP or NAD group compound, and
$A_2$ is an oxidized form of $A_1$.

**Patentansprüche**

**1.** Verfahren zum Testen einer Probe auf Myo-inositol, bei dem man die Probe umsetzt mit:

a) Myo-inositol-Dehydrogenase unter Verwendung eines Coenzyms der Thionicotinamid-adenindinucleotidphosphat-Gruppe (Thio-NADP-Gruppe) oder Thionicotinamid-adenindiculeotid-Gruppe (Thio-NAD-Gruppe) und eines Coenzyms der Nicotinamidadenindinucleotidphosphat-Gruppe (NADP-Gruppe) oder Nicotinamidadenindinucleotid-Gruppe (NAD-Gruppe), wobei die Myo-inositol-Dehydrogenase eine reversible Reaktion unter Bildung von Myo-inosose aus Myo-inositol katalysiert;
b) $A_1$; und
c) $B_1$; und

die cyclische Reaktion durchführt,

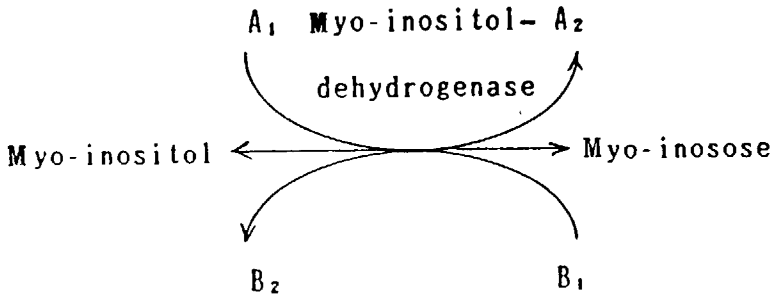


worin

$A_1$ eine Verbindung der Thio-NADP-, Thio-NAD-, NADP- oder NAD-Gruppe ist,
$A_2$ eine reduzierte Form von $A_1$ ist,
$B_1$ eine reduzierte Verbindung der NADP- oder NAD-Gruppe ist, wenn $A_1$ eine Verbindung der Thio-NADP- oder Thio-NAD-Gruppe ist, oder
$B_1$ eine reduzierte Verbindung der Thio-NADP- oder Thio-NAD-Gruppe ist, wenn $A_1$ eine Verbindung der NADP- oder NAD-Gruppe ist, und
$B_2$ eine oxidierte Form von $B_1$ ist, die Menge an $A_2$ oder $B_1$ mißt, die bei der cyclischen Reaktion erzeugt oder verbraucht wird.

**2.** Verfahren zum Testen einer Probe auf Myo-inositol, bei dem man die Probe umsetzt mit:

a) Myo-inositol-Dehydrogenase, wobei man ein Coenzym der Thio-NADP-Gruppe oder der Thio-NAD-Gruppe und ein Coenzym der NADP- oder der NAD-Gruppe verwendet, wobei die Myo-inositol-Dehydrogenase eine reversible Reaktion unter Bildung von Myo-inosose aus Myo-inositol katalysiert;
b) $A_1$;
c) $B_1$ oder/und $B_2$; und
d) einer Dehydrogenase (Dehydrogenase X), die nicht auf Myo-inositol einwirkt und die eine Umsetzung unter Bildung von $B_1$ aus B2 katalysiert,
und einem Substrat für die Dehydrogenase X;
und die cyclische Reaktion durchführt,

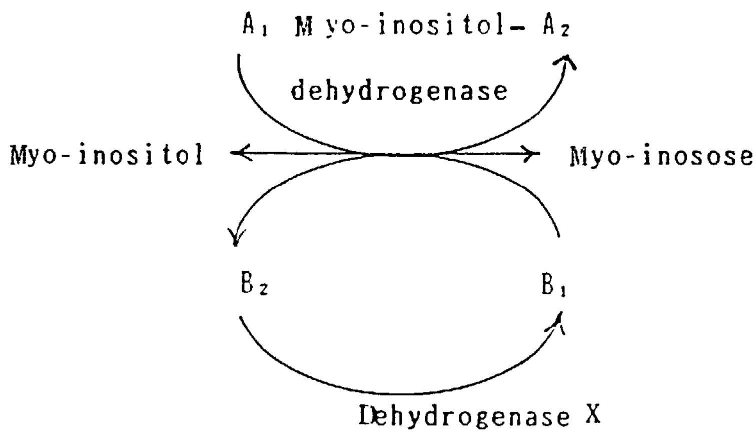


worin

$A_1$ eine Verbindung Thio-NADP-, Thio-NAD-, NADP- oder NAD-Gruppe ist,
$A_2$ eine reduzierte Form von $A_1$ ist,
$B_1$ eine reduzierte Verbindung der NADP- oder NAD-Gruppe ist, wenn $A_1$ eine Verbindung der Thio-NADP- oder Thio-NAD-Gruppe ist, oder
$B_1$ eine reduzierte Verbindung der Thio-NADP- oder Thio-NAD-Gruppe ist, wenn $A_1$ eine Verbindung der NADP- oder NAD-Gruppe ist, und

$B_2$ eine oxidierte Form von $B_1$ ist,
wobei die Reaktion von $B_2$ zu $B_1$ eine enzymatische Reaktion ist, die $B_1$ aus $B_2$ durch die Wirkung der Dehydrogenase X mit einem Coenzym erzeugt; und
die Menge an $A_2$ mißt, das bei der cyclischen Reaktion gebildet wird.

**3.** Verfahren zum Testen einer Probe auf Myo-inositol, bei dem man die Probe umsetzt mit:

a) Myo-inositol-Dehydrogenase, wobei man ein Coenzym der Thio-NADP-Gruppe oder Thio-NAD-Gruppe und ein Coenzym der NADP-Gruppe oder NAD-Gruppe verwendet, wobei die Myo-inositol-Dehydrogenase eine reversible Reaktion unter Bildung von Myo-inosose aus Myo-inositol katalysiert,
b) $A_1$ oder/und $A_2$,
c) $B_1$ und
d) einer Dydrogenase (Dehydrogenase Y), die nicht auf Myo-inositol einwirkt und die eine Reaktion von $A_2$ zu $A_1$
katalysiert, und einem Substrat für die Dehydrogenase Y; und die cyclische Reaktion durchführt,

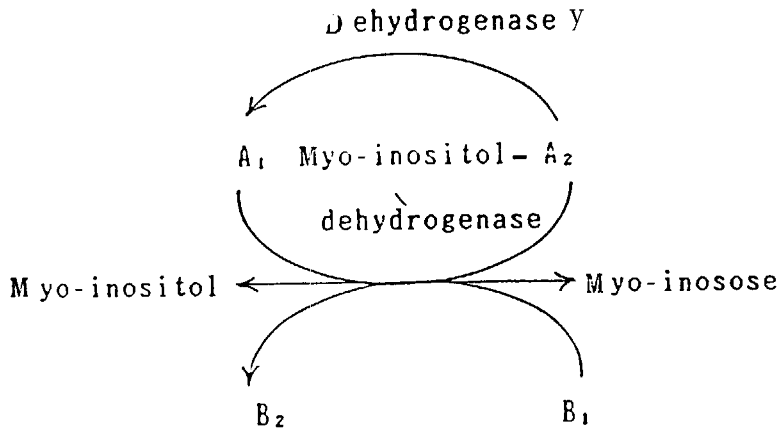


worin

$A_1$ eine Verbindung der Thio-NADP-, Thio-NAD-, NADP- oder NAD-Gruppe ist,
A2 eine reduzierte Form von $A_1$ ist, $B_1$ eine reduzierte Verbindung der NADP- oder NAD-Gruppe ist, wenn $A_1$ eine Verbindung der NADP- oder NAD-Gruppe ist, und
B2 eine oxidierte Form von $B_1$ ist,
wobei die Reaktion von $A_2$ zu $A_1$ eine enzymatische Reaktion ist, die $A_1$ aus $A_2$ durch Wirkung der Dehydrogenase Y mit einem Coenzym bildet; und
die Menge an $B_1$ mißt, die bei der cyclischen Reaktion verbraucht wird.

**4.** Verfahren nach einem der vorhergehenden Ansprüche, wobei es sich bei der Verbindung der Thio-NADP-Gruppe um Thionicotinamidadenindinucleotid-phosphat (Thio-NADP) oder Thionicotinamidhypoxanthin-dinucleotidphosphat handelt.

**5.** Verfahren nach einem der Ansprüche 1 bis 3, bei dem die Verbindung der Thio-NAD-Gruppe Thionicotinamid-adenindinucleotid (Thio-NAD) oder Thionicotinamid-hypoxanthindinucleotid ist.

**6.** Verfahren nach einem der vorhergehenden Ansprüche, bei dem die Verbindung der NADP-Gruppe Nicotinamidadenindinucleotidphosphat (NADP), Acetylpyridin-adenindinucleotidphosphat (Acetyl-NADP) oder Nicotinamid-hypoxanthindinucleotidphosphat (Deamino-NADP) ist.

**7.** Verfahren nach einem der Ansprüche 1 bis 5, bei dem die Verbindung der NADP-Gruppe Nicotinamid-adenindinucleotid (NAD), Acetylpyridin-adenindinucleotidphosphat (Acetyl-NAD) oder Nicotinamid-hypoxanthindinucleotidphosphat (Deamino-NAD) ist.

**8.** Reagens-Zusammensetzung zum Testen von Myo-inositol, umfassend:

a) Myo-inositol-Dehydrogenase unter Verwendung eines Coenzyms der Thionicotinamid-adenindinucleotidphosphat-Gruppe (Thio-NADP-Gruppe) oder Thionicotinamid-adenindinucleotid-Gruppe (Thio-NAD-Gruppe) und eines Coenzyms der Nicotinamidadenindinucleotidphosphat-Gruppe (NADP-Gruppe) oder Nicotinamidadenindinucleotid-Gruppe (NAD-Gruppe), wobei die Myo-inositol-Dehydrogenase eine reversible Reaktion unter Bildung von Myo-inosose aus Myo-inositol katalysiert;
b) $A_1$; und
c) $B_1$;

worin

$A_1$ eine Verbindung der Thio-NADP-, Thio-NAD-, NADP- oder NAD-Gruppe ist,
$B_1$ eine reduzierte Verbindung der NADP- oder NAD-Gruppe ist, wenn $A_1$ eine Verbindung der Thio-NADP- oder Thio-NAD-Gruppe ist, oder
$B_1$ eine reduzierte Verbindung der Thio-NADP- oder Thio-NAD-Gruppe ist, wenn $A_1$ eine Verbindung der NADP- oder NAD-Gruppe ist.

**9.** Reagens-Zusammensetzung zum Testen von Myo-inositol, umfassend:

a) Myo-inositol-Dehydrogenase unter Verwendung eines Coenzyms der Thio-NADP-Gruppe oder Thio-NAD-Gruppe oder eines Coenzyms der NADP-Gruppe oder NAD-Gruppe, wobei die Myo-inositol-Dehydrogenase eine reversible Reaktion unter Bildung von Myo-inosose aus Myo-inositol katalysiert;
b) $A_1$;
c) $B_1$ oder/und B2; und
d) eine Dehydrogenase (Dehydrogenase X), die nicht auf Myo-inositol einwirkt und eine Reaktion unter Bildung von $B_1$ aus B2 katalysiert, und ein Substrat für die Dehydrogenase X; worin
$A_1$ eine Verbindung der Thio-NADP-, Thio-NAD-, NADP- oder NAD-Gruppe ist,
$B_1$ eine reduzierte Verbindung der NADP- oder NAD-Gruppe ist, wenn $A_1$ eine Verbindung der Thio-NADP- oder Thio-NAD-Gruppe ist, oder
$B_1$ eine reduzierte Verbindung der Thio-NADP- oder Thio-NAD-Gruppe ist, wenn $A_1$ eine Verbindung der NADP- oder NAD-Gruppe ist, und
B2 eine oxidierte Form von $B_1$ ist.

**10.** Reagens-Zusammensetzung zum Testen von Myo-inositol, umfassend:

a) Myo-inositol-Dehydrogenase unter Verwendung eines Coenzyms der Thio-NADP-Gruppe oder Thio-NAD-Gruppe und eines Coenzyms der NADP-Gruppe oder NAD-Gruppe, wobei die Myo-inositol-Dehydrogenase eine reversible Reaktion unter Bildung von Myo-inosose aus Myo-inositol katalysiert;
b) $A_1$ oder/und $A_2$;
c) $B_1$; und
d) eine Dehydrogenase (Dehydrogenase Y), die nicht auf Myo-inositol einwirkt und eine Reaktion von $A_2$ zur $A_1$ katalysiert, und ein Substrat für die Dehydrogenase Y; worin
$A_1$ eine Verbindung der Thio-NADP-, Thio-NAD-, NADP- oder NAD-Gruppe ist,
$B_1$ eine reduzierte Verbindung der NADP- oder NAD-Gruppe ist, wenn $A_1$ eine Verbindung der Thio-NADP- oder Thio-NAD-Gruppe ist, oder
$B_1$ eine reduzierte Verbindung der Thio-NADP- oder Thio-NAD-Gruppe ist, wenn $A_1$ eine Verbindung der NADP- oder NAD-Gruppe ist, und
$A_2$ eine oxidierte Form von $A_1$ ist.

## Revendications

**1.** Méthode de dosage d'un spécimen à la recherche du myo-inositol qui comprend la réaction du spécimen avec :

a) la myo-inositol déshydrogénase en utilisant l'un du groupe thionicotinamide adénine dinucléotide phosphate (groupe thio-NADP) ou du groupe thionicotinamide adénine dinucléotide (groupe thio-NAD) et l'un du groupe nicotinamide adénine dinucléotide phosphate (groupe NADP) ou du groupe nicotinamide adénine dinucléotide (groupe NAD) comme coenzymes, laquelle myo-inositol déshydrogénase catalyse une réaction réversible formant du myo-inosose à partir de myo-inositol,

b) $A_1$, et
c) $B_1$;

pour effectuer la réaction de cycle :

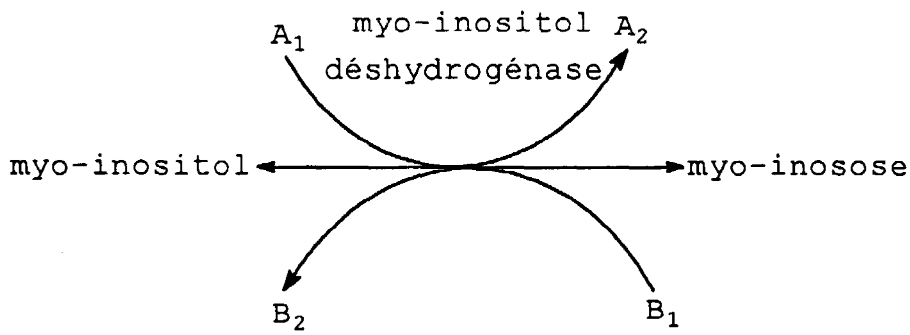


où :

$A_1$ est un composé du groupe thio-NADP, thio-NAD, NADP ou NAD,
$A_2$ est une forme réduite de $A_1$,
$B_1$ est un composé du groupe NADP réduit ou NAD réduit quand $A_1$ est un composé du groupe thio-NADP ou thio-NAD ou $B_1$ est un composé du groupe thio-NADP réduit ou thio-NAD réduit quand $A_1$ est un composé du groupe NADP ou NAD, et
$B_2$ est une forme oxydée de $B_1$; et
la mesure de la quantité de $A_2$ ou $B_1$ généré ou consommé dans la réaction de cycle.

**2.** Méthode de dosage d'un spécimen à la recherche du myo-inositol qui comprend la réaction du spécimen avec :

a) la myo-inositol déshydrogénase en utilisant l'un du groupe thio-NADP ou du groupe thio-NAD et l'un du groupe NADP ou du groupe NAD comme coenzymes, laquelle myo-inositol déshydrogénase catalyse une réaction réversible formant le myo-inosose à partir du myo-inositol,
b) $A_1$,
c) $B_1$ et/ou $B_2$, et
d) une déshydrogénase (déshydrogénase X) qui n'agit pas sur le myo-inositol et qui catalyse une réaction formant $B_1$ à partir de $B_2$, et un substrat pour ladite déshydrogénase X;

pour effectuer la réaction de cycle :

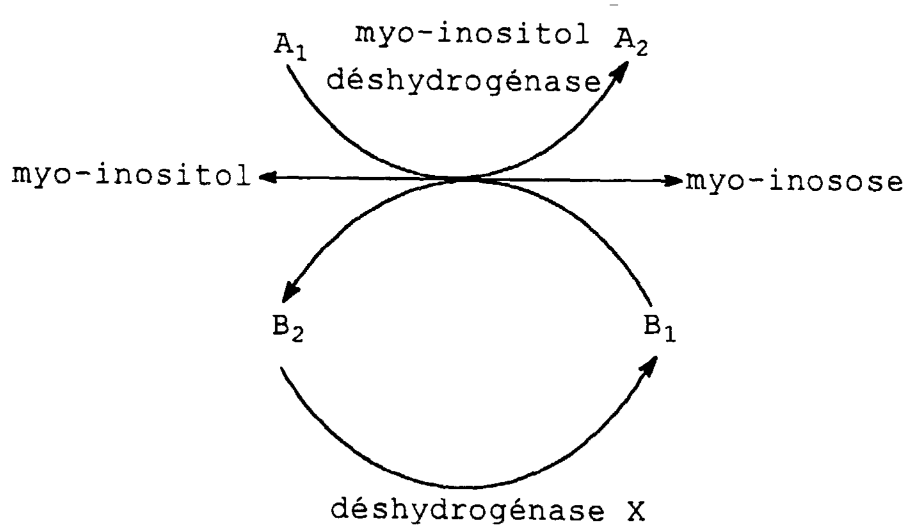


où :

$A_1$ est un composé du groupe thio-NADP, thio-NAD, NADP ou NAD,
$A_2$ est une forme réduite de $A_1$,
$B_1$ est un composé du groupe NADP réduit ou NAD réduit quand $A_1$ est un composé du groupe thio-NADP

ou thio-NAD ou $B_1$ est un composé du groupe thio-NADP réduit ou thio-NAD réduit quand $A_1$ est un composé du groupe NADP ou NAD, et
$B_2$ est une forme oxydée de $B_1$;
la réaction de $B_2$ à $B_1$ étant une réaction enzymatique qui génère $B_1$ à partir de $B_2$ par une action de la déshydrogénase X avec une coenzyme; et
la mesure de la quantité de $A_2$ produit dans la réaction de cycle.

**3.** Méthode de dosage d'un spécimen à la recherche du myo-inositol qui comprend la réaction du spécimen avec :

a) la myo-inositol déshydrogénase en utilisant l'un du groupe thio-NADP ou thio-NAD et l'un du groupe NADP ou du groupe NAD comme coenzymes, laquelle myo-inositol déshydrogénase catalyse une réaction réversible formant le myo-inosose à partir du myo-inositol,
b) $A_1$ et/ou $A_2$,
c) $B_1$ et
d) une déshydrogénase (déshydrogénase Y) qui n'agit pas sur le myo-inositol et qui catalyse une réaction de $A_2$ à $A_1$, et un substrat pour ladite déshydrogénase Y;

pour effectuer la réaction de cycle :

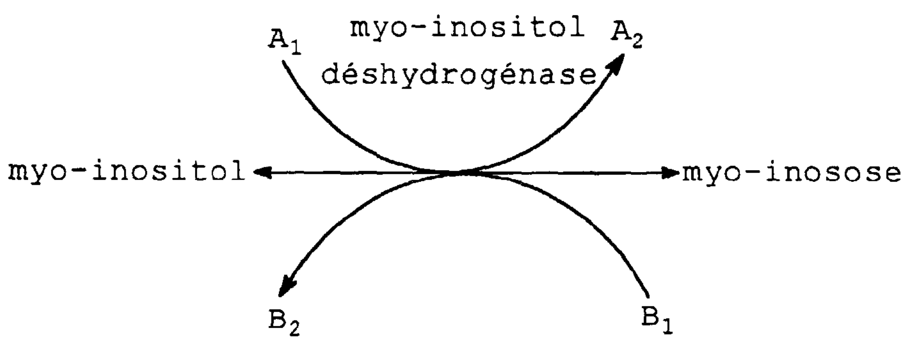


où :

$A_1$ est un composé du groupe thio-NADP, thio-NAD, NADP ou NAD,
$A_2$ est une forme réduite de $A_1$,
$B_1$ est un composé du groupe NADP réduit ou NAD réduit quand $A_1$ est composé du groupe NADP ou NAD, et
$B_2$ est une forme oxydée de $B_1$;
la réaction de $A_2$ à $A_1$ étant une réaction enzymatique qui produit $A_1$ à partir de $A_2$ par une action de la déshydrogénase Y avec une coenzyme; et
la mesure de la quantité de $B_1$ consommé dans la réaction de cycle.

**4.** Méthode selon l'une quelconque des revendications précédentes où le groupe thio-NADP est le thionicotinamide adénine dinucléotide phosphate (thio-NADP) ou thionicotinamide hypoxanthine dinucléotide phosphate.

**5.** Méthode selon l'une quelconque des revendications 1 à 3 où le groupe thio-NAD est le thionicotinamide adénine dinucléotide (thio-NAD) ou le thionicotinamide hypoxanthine dinucléotide.

**6.** Méthode selon l'une quelconque des revendications précédentes où le groupe NADP est le nicotinamide adénine dinucléotide phosphate (NADP), l'acétylpyridine adénine dinucléotide phosphate (acétyl NADP) ou le nicotinamide hypoxanthine dinucléotide phosphate (déamino-NADP).

**7.** Méthode selon l'une quelconque des revendications 1 à 5 où le groupe NAD est le nicotinamide adénine dinucléotide (NAD), l'acétylpyridine adénine dinucléotide phosphate (acétyl NAD), ou le nicotinamide hypoxanthine dinucléotide phosphate (déamino-NAD).

**8.** Composition de réactifs appropriée au dosage du myo-inositol qui comprend :

a) de la myo-inositol déshydrogénase utilisant l'un du thionicotinamide adénine dinucléotide phosphate (groupe thio-NADP) ou du groupe thionicotinamide adénine dinucléotide (groupe thio-NAD) et l'un du groupe nicotinamide adénine dinucléotide phosphate(groupe NADP) ou du groupe nicotinamide dinucléotide (groupe NAD) comme coenzymes, laquelle myo-inositol déshydrogénase catalyse une réaction réversible formant le myo-inosose à partir du myo-inositol,
b) $A_1$ et
c) $B_1$;

où :

$A_1$ est un composé du groupe thio-NADP, thio-NAD, NADP ou NAD,
$B_1$ est un composé du groupe NADP réduit ou NAD réduit quand $A_1$ est un composé thio-NADP ou thio-NAD ou $B_1$ est un composé du groupe thio-NADP réduit ou thio-NAD réduit quand $A_1$ est un composé du groupe NADP ou NAD.

**9.** Composition de réactifs appropriés au dosage du myo-inositol qui comprend :

a) de la myo-inositol déshydrogénase utilisant l'un du groupe thio-NADP ou du groupe thio-NAD et l'un du groupe NADP ou du groupe NAD comme coenzymes, laquelle myo-inositol déshydrogénase catalyse une réaction réversible formant le myo-inosose à partir du myo-inositol,
b) $A_1$,
c) $B_1$ et/ou $B_2$, et
d) une déshydrogénase (déshydrogénase X) qui n'agit pas sur le myo-inositol et qui catalyse une réaction formant $B_1$ à partir de $B_2$, et un substrat pour ladite déshydrogénase X,

où :

$A_1$ est un composé du groupe thio-NADP, thio-NAD, NADP ou NAD,
$B_1$ est un composé du groupe NADP réduit ou NAD réduit quand $A_1$ est un composé du groupe thio-NADP ou thio-NAD ou $B_1$ est un composé du groupe thio-NADP réduit ou thio-NAD réduit quand $A_1$ est un composé du groupe NADP ou NAD, et
$B_2$ est une forme oxydée de $B_1$.

**10.** Composition de réactifs approprié au dosage du myo-inositol qui comprend :

a) de la myo-inositol déshydrogénase utilisant l'un du groupe thio-NADP ou du groupe thio-NAD et l'un du groupe NADP ou du groupe NAD comme coenzymes, laquelle myo-inositol déshydrogénase catalyse une réaction réversible formant le myo-inosose à partir du myo-inositol,
b) $A_1$ et/ou $A_2$
c) $B_1$ et
d) une déshydrogénase (déshydrogénase Y) qui n'agit pas sur le myo-inositol et qui catalyse une réaction de $A_2$ à $A_1$, et un substrat de ladite déshydrogénase Y;

où :

$A_1$ est un composé du groupe thio-NADP, thio-NAD, NADP ou NAD,
$B_1$ est un composé du groupe NADP réduit ou NAD réduit quand $A_1$ est un composé du groupe thio-NADP ou thio-NAD ou $B_1$ est un composé du groupe thio-NADP réduit ou thio-NAD réduit quand $A_1$ est un composé du groupe NADP ou NAD, et
$A_2$ est une forme oxydée de $A_1$.

FIG. 1

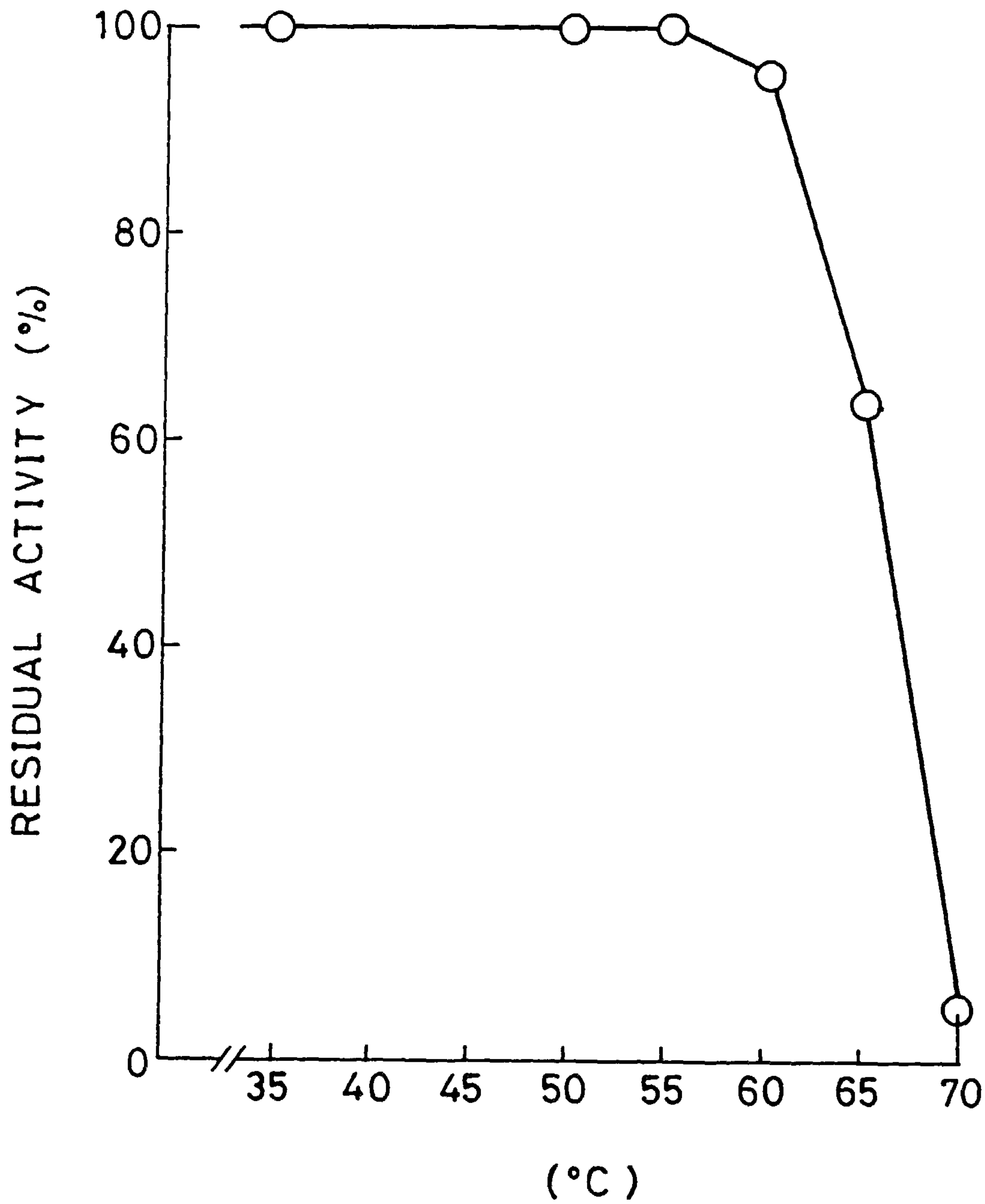

FIG. 2

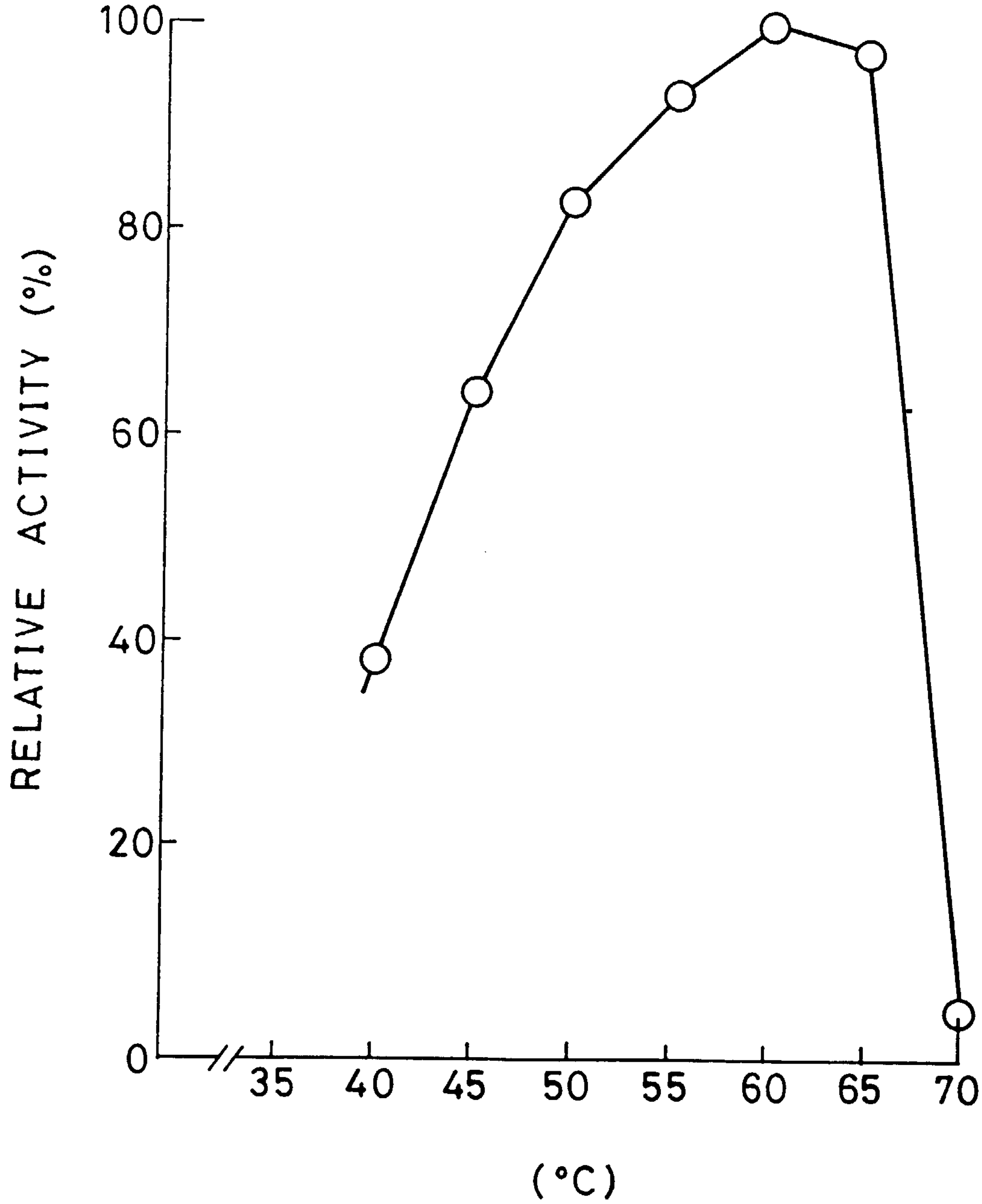

FIG. 3

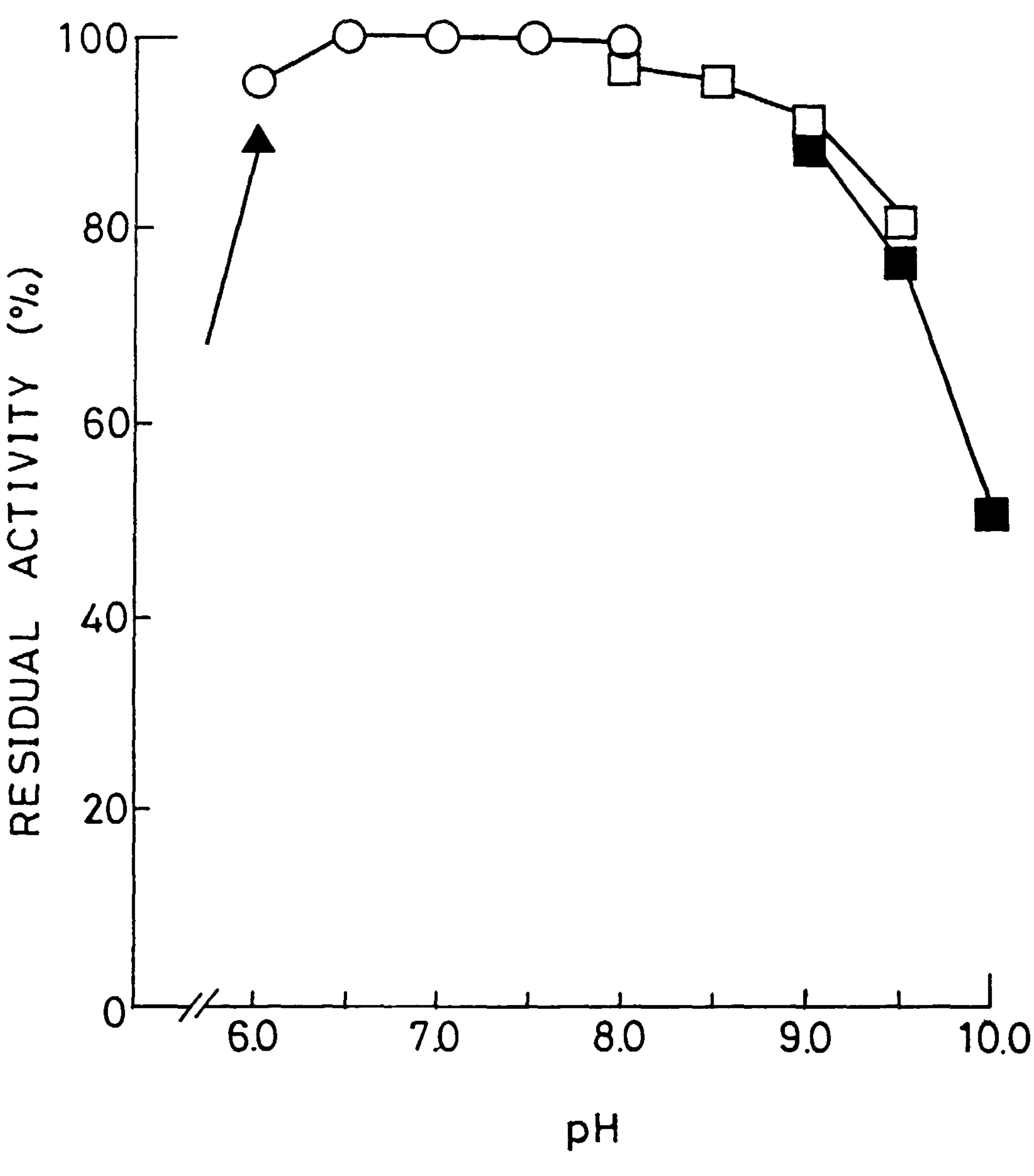

FIG. 4

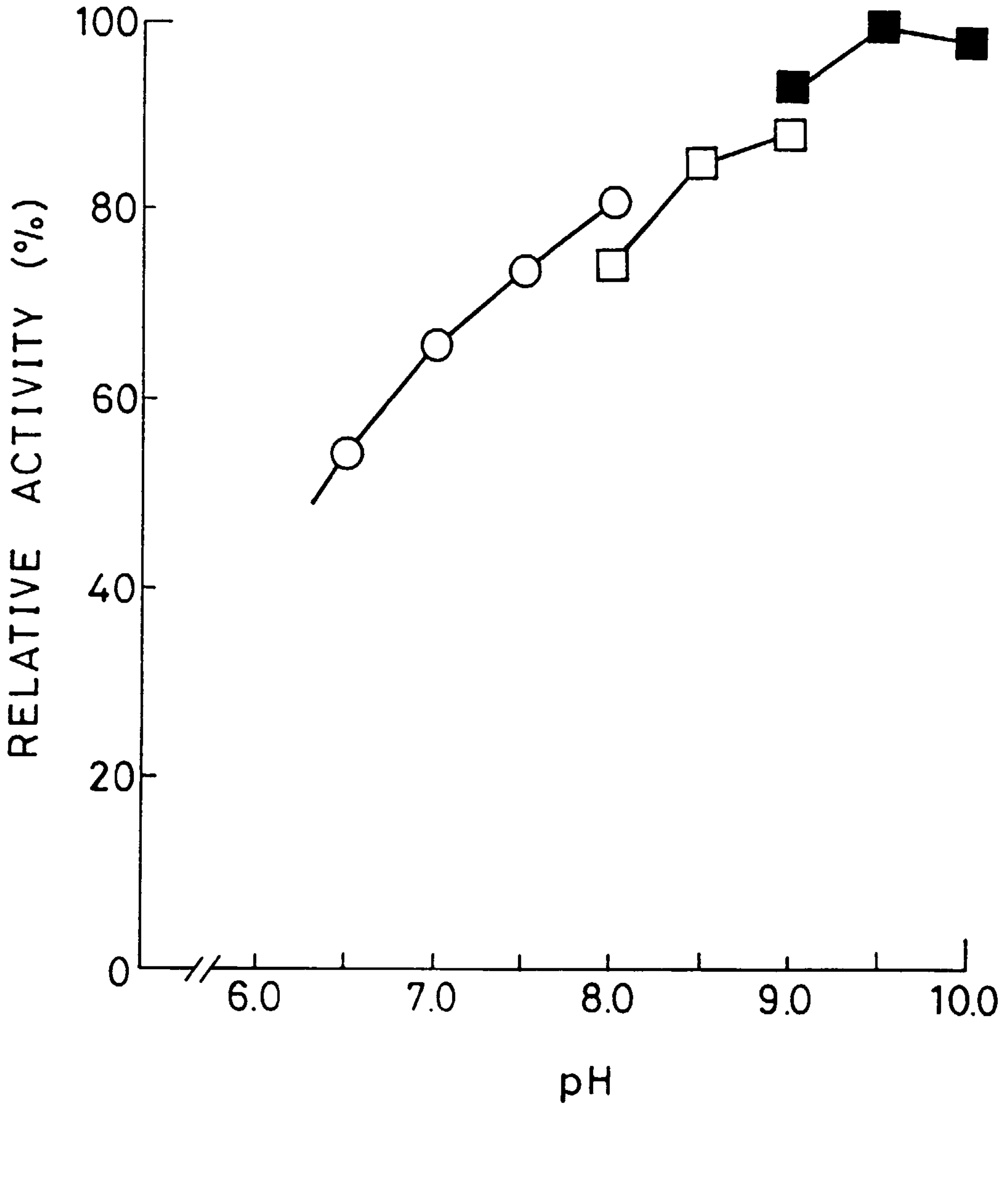

FIG. 5

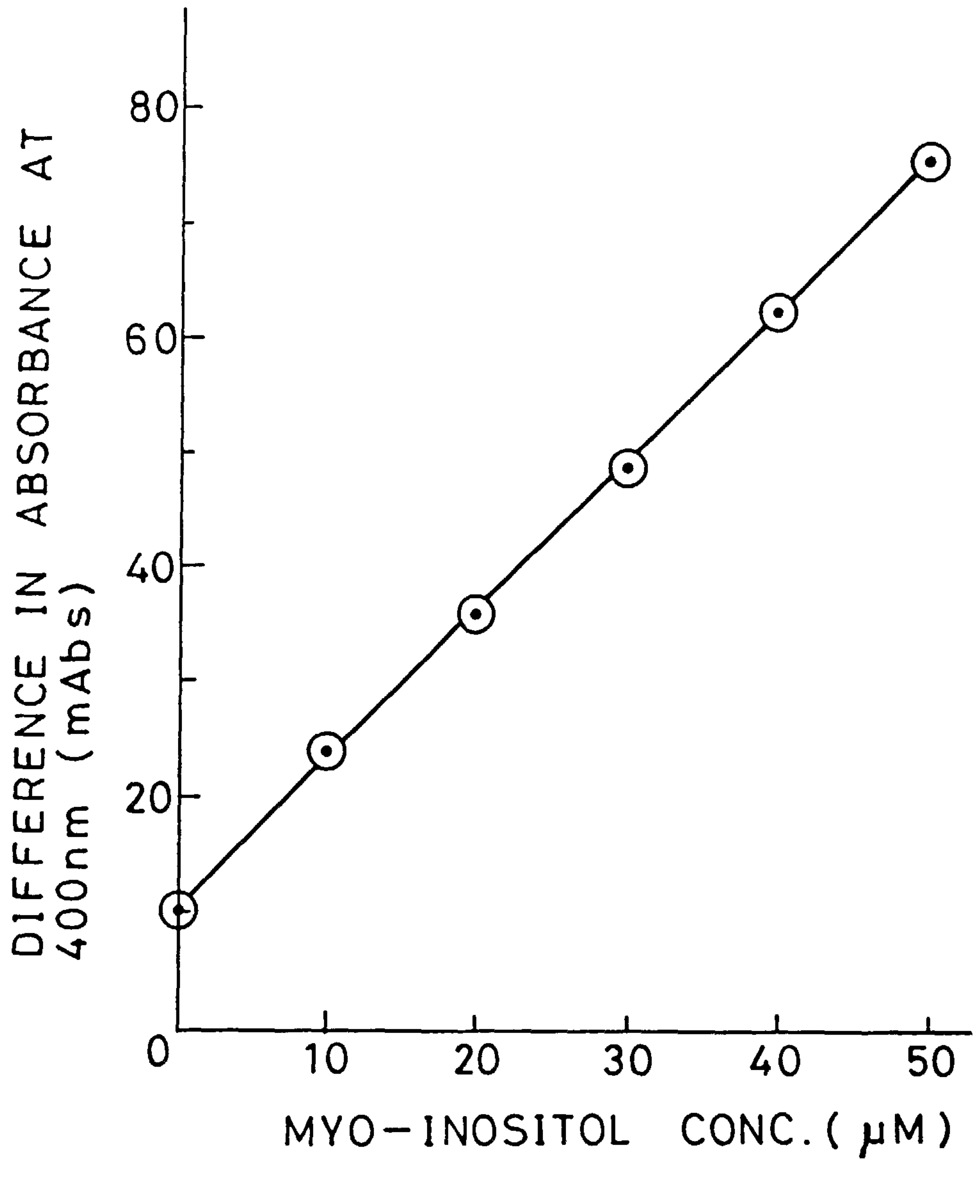
DIFFERENCE IN ABSORBANCE AT 400nm (mAbs)
80
60
40
20
0
10
20
30
40
50
MYO-INOSITOL CONC.( μM)

FIG. 6

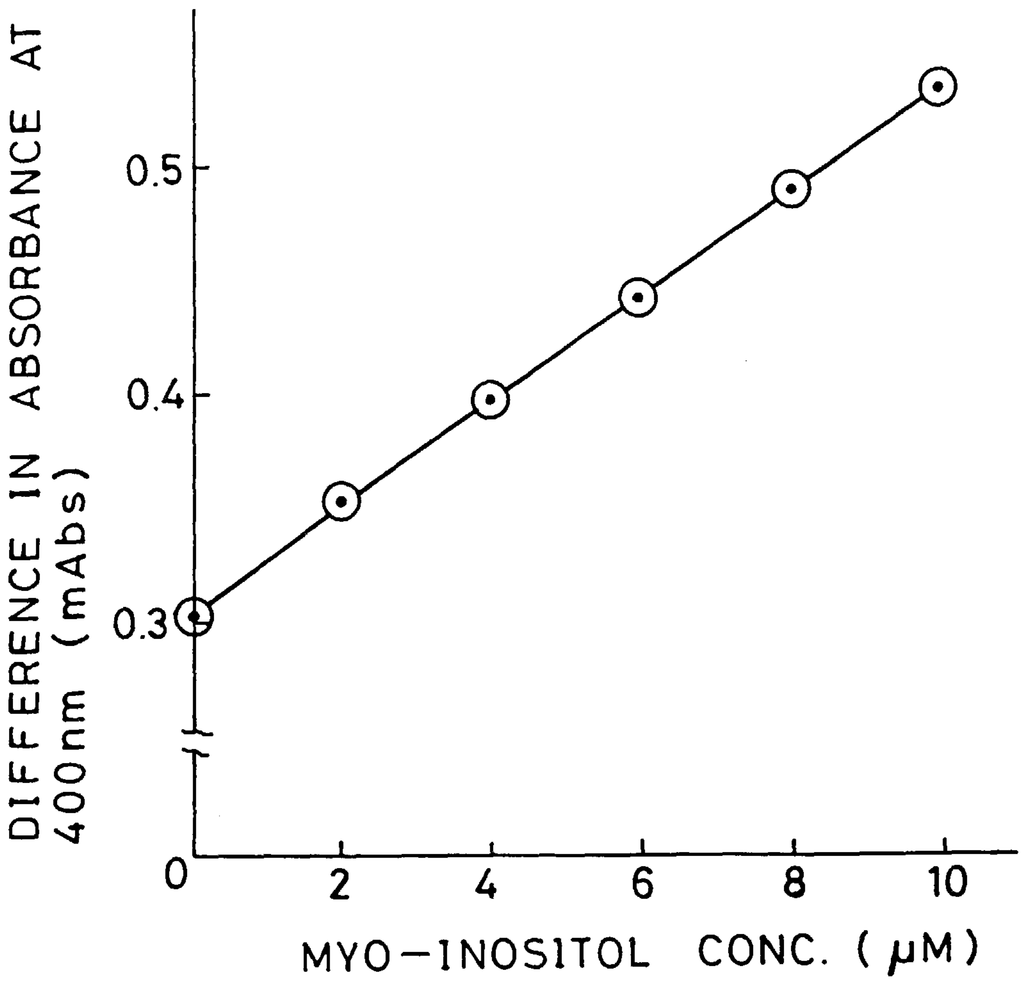
DIFFERENCE IN ABSORBANCE AT 400nm (mAbs)
0.5
0.4
0.3
0
2
4
6
8
10
MYO-INOSITOL CONC. (μM)

FIG. 7

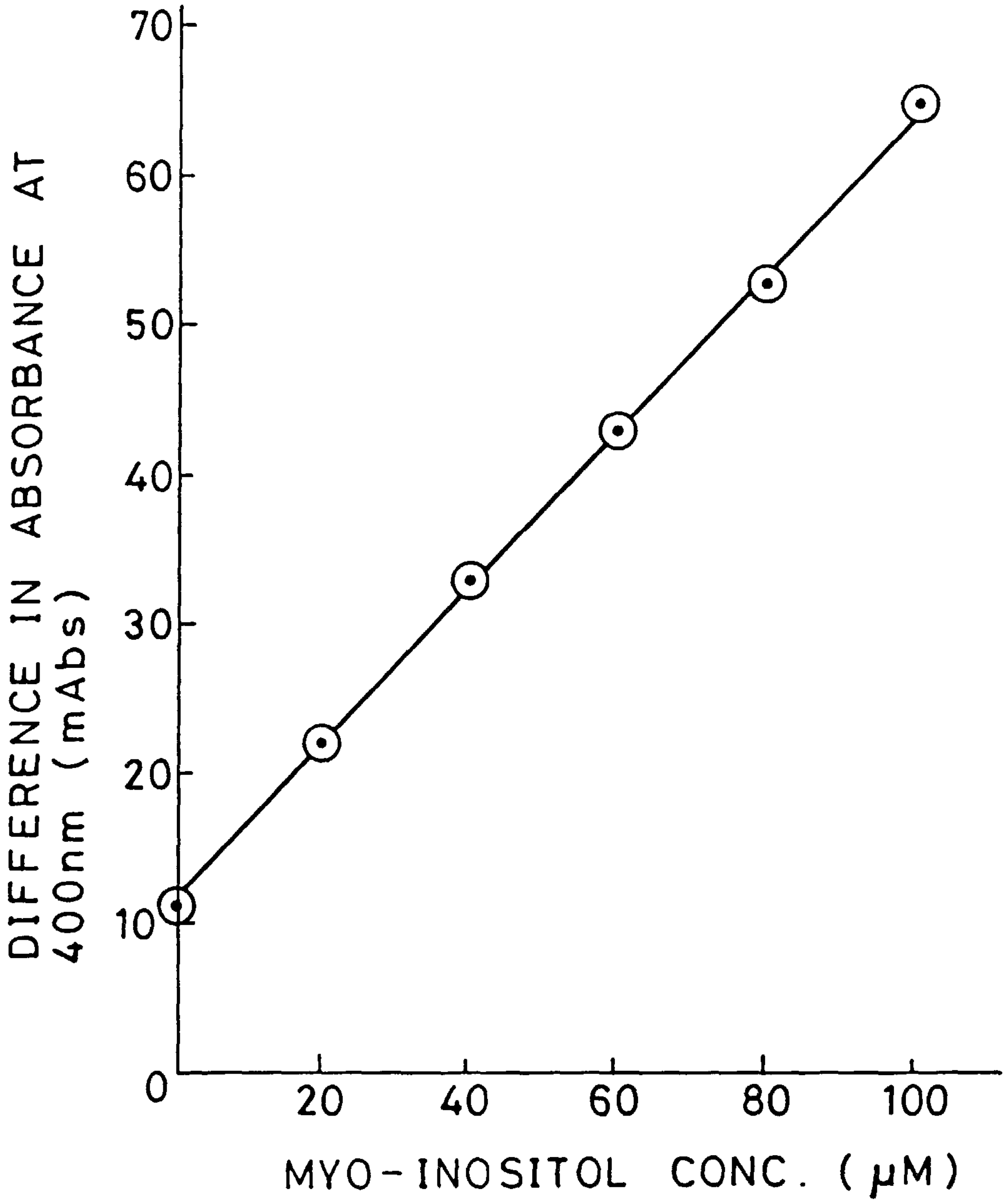
70
60
50
40
30
20
10
0
20
40
60
80
100
DIFFERENCE IN ABSORBANCE AT 400nm (mAbs)
MYO-INOSITOL CONC. (μM)

FIG. 8

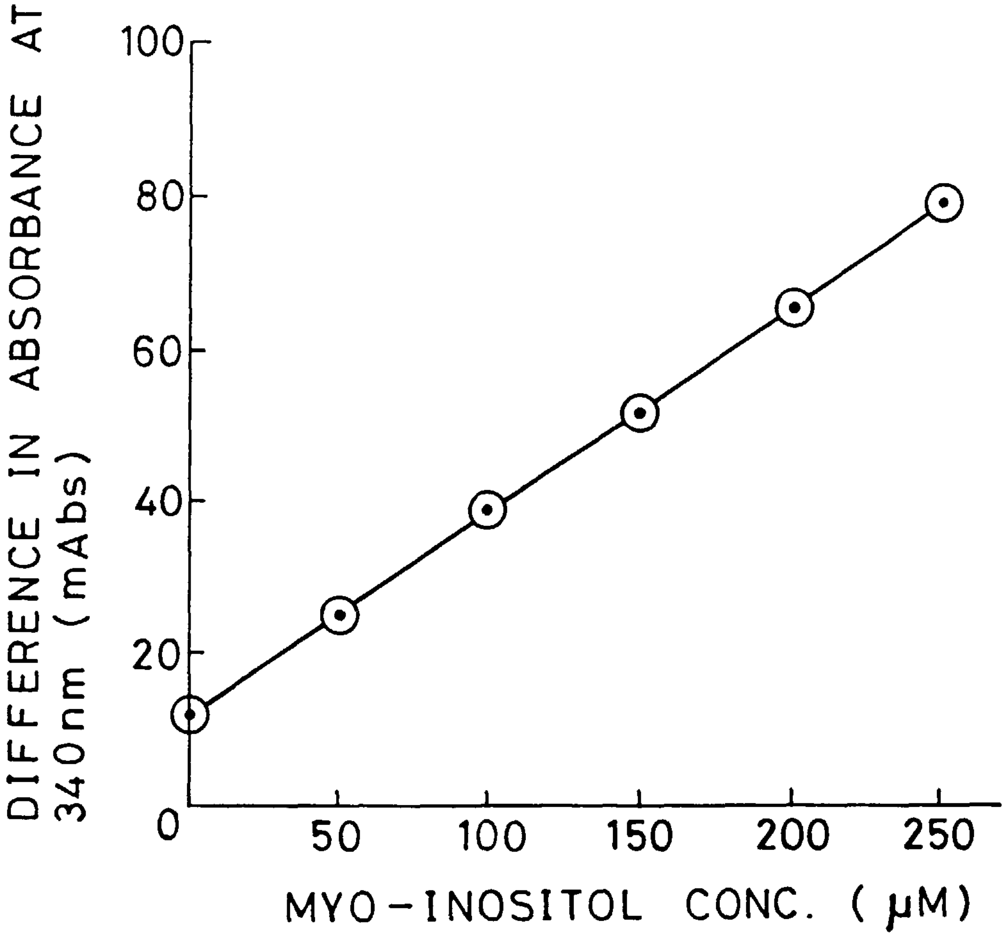
DIFFERENCE IN ABSORBANCE AT 340nm (mAbs)
100
80
60
40
20
0
50
100
150
200
250
MYO-INOSITOL CONC. (μM)